# EUROPEAN PATENT APPLICATION

(11) **EP 3 868 748 A1**
(43) Date of publication of application: **25.08.2021**
(21) Application number: 19872630.9
(22) Date of filing: 15.10.2019
(51) Int. Cl.: C07D 401/14, C09K 11/06, H01L 51/50

(54) **COMPOUND HAVING PYRIMIDINE RING STRUCTURE AND ORGANIC ELECTROLUMINESCENT ELEMENT**

(30) Priority: 17.10.2018 JP 2018195569
(71) Applicant: Hodogaya Chemical Co., Ltd., Tokyo 104-0028 (JP)
(72) Inventor: KASE, Kouki, Tokyo 104-0028 (JP); KIM, Si-In, Tokyo 104-0028 (JP); HIRAYAMA, Yuta, Tokyo 104-0028 (JP)
(74) Representative: Forstmeyer, Dietmar
(86) International application number: PCT/JP2019/040409
(87) International publication number: WO 2020/080341

(57) **Abstract**

An object of the present invention is to provide, as a material for a highly efficient and highly durable organic EL element, an organic compound having excellent properties, including excellent electron-injecting/transporting capability, hole-blocking capability, and high stability in the form of a thin film. Another object of the present invention is to provide a highly efficient and highly durable organic EL element by using this compound. The present invention provides a compound having a pyrimidine ring structure that is designed and chemically synthesized with the focus on the properties of the pyrimidine ring, which has affinity for electrons, specifically with the focus on the capability of the nitrogen atoms to coordinate to a metal and excellent heat resistance. Various organic EL elements including the compound were experimentally produced, followed by evaluation of the characteristics of those elements. From the results, it was found that the organic EL elements have favorable characteristics.

## Description

### Technical Field

The present invention relates to a compound suitable for organic electroluminescent elements (hereinafter referred to simply as "organic EL elements"), which are self-light-emitting elements favorably used in various display devices, and also to an organic EL element. More particularly, the present invention relates to a compound having a pyrimidine ring structure and an organic EL element including the compound.

### Background Art

Since organic EL elements are self-emissive elements, they have larger brightness and better view ability than elements including liquid crystals, and can thus provide a clearer display. For these reasons, active studies have been carried out on organic EL elements.

In 1987, C. W. Tang et al. of Eastman Kodak Company developed an element having a layered structure in which various functions were assigned to different materials, and thus made a practical organic EL element including organic materials. They made an organic EL element having a layered structure including a layer of a fluorescent substance capable of transporting electrons and a layer of an organic substance capable of transporting holes, and injected both charges into the layer of the fluorescent substance to thereby cause the layer to emit light, and the organic EL element thus achieved a luminance as high as 1,000 cd/m² or more at a voltage of 10 V or less (see Patent Literatures 1 and 2, for example).

Organic EL elements have been heretofore much improved to put them to practical use. Electroluminescent elements have been suggested in which an anode, a hole-injecting layer, a hole-transporting layer, a light-emitting layer, an electron-transporting layer, an electron-injecting layer, and a cathode are sequentially provided on a substrate to subdivide various functions in the multi-layered structure even further, and such electroluminescent elements successfully have high efficiency and durability (see Non-Patent Literature 1, for example).

To further increase luminous efficacy, attempts have been made to utilize triplet excitons, and the utilization of phosphorescent compounds has been investigated (see Non-Patent Literature 2, for example).

Moreover, elements that utilize light emission by thermally activated delayed fluorescence (TADF) have also been developed. In 2011, Adachi et al. from Kyushu University achieved a result of an external quantum efficiency of 5.3% by an element including a thermally activated delayed fluorescence material (see Non-Patent Literature 3, for example).

The light-emitting layer can also be prepared by doping a charge-transporting compound, generally called a host material, with a fluorescent compound, a phosphorescent compound, or a material that radiates delayed fluorescence. As stated in the non-patent literature above, the selection of the organic materials in an organic EL element greatly affects the characteristics of that element, such as efficiency and durability (see Non-Patent Literature 2, for example).

In an organic EL element, the charges injected from both electrodes recombine in the light-emitting layer, thereby producing light emission, and how efficiently the both charges, i.e., the holes and the electrons, are passed to the light-emitting layer is of importance. For this purpose, the probability that holes and electrons recombine in the light-emitting layer can be increased by enhancing the electron-injecting capability and increasing the electron mobility. Furthermore, an environment that increases the probability of the recombination even more can be created by confining holes transported from the anode side in the light-emitting layer, preventing deterioration of the electron-transporting layer, and confining excitons generated in the light-emitting layer, and highly efficient light emission can thus be achieved. Therefore, the functions of the electron-transporting material are important, and there is a need for an electron-transporting material that has great electron-injecting capability, high electron mobility, high hole-blocking capability, and high durability against holes.

Moreover, with regard to element lifespan, heat resistance and amorphousness of the materials are also important. A material with low heat resistance thermally decomposes, due to heat generated during driving the element, even at a low temperature, and thus the material deteriorates. A film made of a material with low amorphousness causes crystallization thereof even in a short period of time to result in deterioration of the element. Thus, the materials to be used are required to have high heat resistance and good amorphousness.

Tris(8-hydroxyquinoline)aluminum (hereinafter abbreviated as Alq3), which is a typical light emitting material, is also commonly used as an electron-transporting material; however, it provides low electron mobility and has a work function of 5.6 eV, and therefore it cannot be said that Alq3 has sufficient hole-blocking capability.

Compounds having a benzotriazole structure have been suggested as compounds improved in the properties including the electron-injecting capability and the electron mobility (see Patent Literature 3, for example). Elements having an electron-transporting layer including such a compound have the improved properties including luminous efficacy; however, these properties are still insufficient. Therefore, there is a demand for a further decrease in driving voltage and a further increase in luminous efficacy.

Also, 3-(4-biphenylyl)-4-phenyl-5-(4-t-butylphenyl)-1,2,4-triazole (hereinafter abbreviated as TAZ) has been suggested as an electron-transporting material with excellent hole-blocking capability (see Patent Literature 4, for example).

TAZ has a work function as high as 6.6 eV and has high hole-blocking capability. Therefore, TAZ is used for an electron transportable, hole-blocking layer stacked on the cathode side of a fluorescent or phosphorescent light-emitting layer produced by vacuum deposition or coating, and TAZ thus contributes to an increase in the efficiency of an organic EL element (see Non-Patent Literature 4, for example).

However, low electron transportability is a critical problem with TAZ, and it is necessary to combine TAZ with an electron-transporting material having higher electron transportability when producing an organic EL element (see Non-Patent Literature 5, for example).

2,9-dimethyl-4,7-diphenyl-1,10-phenanthroline (BCP), which is known as an electron-transporting material, also has a work function as high as 6.7 eV and has high hole-blocking capability. However, BCP has a glass transition point (Tg) as low as 83°C, which results in poor stability of a thin film made of it, and therefore cannot be said to be capable of sufficiently functioning as a hole-blocking layer.

All of these materials have insufficient stability in the form of a film or have insufficient hole-blocking capability. In order to improve characteristics of an organic EL element, there is a demand for an organic compound that has excellent electron-injecting/transporting capability and hole-blocking capability, and also has high stability in the form of a thin film.

### Citation List

### Patent Literature

Patent Literature 1: US 5792557
Patent Literature 2: US 5639914
Patent Literature 3: US 9123897
Patent Literature 4: US 5869199
Patent Literature 5: US 2017/186967
Patent Literature 6: US 9199966
Patent Literature 7: EP 2684932
Patent Literature 8: US 6878469
Patent Literature 9: US 8470454
Patent Literature 10: US 2017/186967

### Non-Patent Literature

Non-Patent Literature 1: Proceedings of the 9th Meeting of the Japan Society of Applied Physics, pp. 55-61 (2001)
Non-Patent Literature 2: Proceedings of the 9th Meeting of the Japan Society of Applied Physics, pp. 23-31 (2001)
Non-Patent Literature 3: Appl. Phys. Let., 98, 083302 (2011)
Non-Patent Literature 4: Extended Abstracts of the 50th Meeting, the Japan Society of Applied Physics and Related Societies, 28p-A-6, p. 1413 (2003)
Non-Patent Literature 5: The Journal of Molecular Electronics and Bioelectronics, the Japan Society of Applied Physics, Vol. 11, No. 1, pp. 13-19 (2000)
Non-Patent Literature 6: J. Org. Chem. 2001, 66, 7125-7128

### Summary of Invention

An object of the present invention is to provide, as a material for a highly efficient and highly durable organic EL element, an organic compound having excellent properties, including excellent electron-injecting/transporting capability, hole-blocking capability, and high stability in the form of a thin film. Furthermore, another object of the present invention is to provide a highly efficient and highly durable organic EL element by using this compound.

An organic compound to be provided by the present invention should have the following physical properties: (1) good electron-injecting properties, (2) high electron mobility, (3) excellent hole-blocking capability, (4) stability in the form of a thin film, and (5) excellent heat resistance. Moreover, an organic EL element to be provided by the present invention should have the following physical characteristics: (1) high luminous efficacy and high power efficiency, (2) a low voltage for the start of light emission, (3) a low driving voltage in actual use, and (4) a long lifespan.

To achieve the above-described objects, the inventors of the present invention have focused on the properties of the pyrimidine ring, which has affinity for electrons, and specifically focused on the capability of its nitrogen atoms to coordinate to a metal and also on excellent heat resistance. The inventors have thus designed and chemically synthesized compounds having a pyrimidine ring structure, and then experimentally produced various organic EL elements including the compounds, followed by thoroughly evaluating the characteristics thereof, and thus, the present invention has been accomplished.
1) Specifically, the present invention provides a compound having a pyrimidine ring structure and represented by the general formula (a-1): 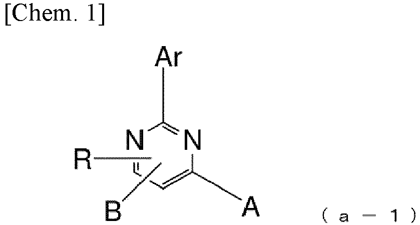 where A and B are the same or different from each other, and each are represented by the structural formula (b-1); Ar represents a substituted or unsubstituted aromatic hydrocarbon group, a substituted or unsubstituted aromatic heterocyclic group, or a substituted or unsubstituted fused polycyclic aromatic group; and R represents a hydrogen atom, a deuterium atom, a fluorine atom, a chlorine atom, a cyano group, a nitro group, a trimethylsilyl group, a triphenylsilyl group, a substituted or unsubstituted aromatic hydrocarbon group, a substituted or unsubstituted aromatic heterocyclic group, a substituted or unsubstituted fused polycyclic aromatic group, a linear or branched alkyl group having 1 to 6 carbon atoms and optionally having a substituent, a cycloalkyl group having 5 to 10 carbon atoms and optionally having a substituent, a linear or branched alkenyl group having 2 to 6 carbon atoms and optionally having a substituent, a linear or branched alkyloxy group having 1 to 6 carbon atoms and optionally having a substituent, or a cycloalkyloxy group having 5 to 10 carbon atoms and optionally having a substituent,
   [Chem. 2]

   **- - - -L-Het** (b - 1)

   where L represents a single bond, or a divalent group of a substituted or unsubstituted aromatic hydrocarbon group, a substituted or unsubstituted aromatic heterocyclic group, or a substituted or unsubstituted fused polycyclic aromatic group; Het represents a substituted or unsubstituted pyridyl group, a substituted or unsubstituted pyrimidyl group, a substituted or unsubstituted triazyl group, a substituted or unsubstituted quinolyl group, a substituted or unsubstituted isoquinolyl group, a substituted or unsubstituted phenanthrolinyl group, a substituted or unsubstituted indolyl group, a substituted or unsubstituted azafluorenyl group, a substituted or unsubstituted diazafluorenyl group, a substituted or unsubstituted azaspirobifluorenyl group, or a substituted or unsubstituted diazaspirobifluorenyl group; and the dashed line represents a binding site to the pyrimidine ring.
2) The present invention also provides the compound having a pyrimidine ring structure as set forth in clause 1), wherein the compound is represented by the general formula (a-2): 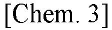 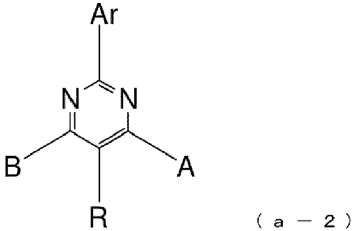 where A, B, Ar, and R are the same as defined in the general formula (a-1).
3) The present invention also provides the compound having a pyrimidine ring structure as set forth in clause 1), wherein the compound is represented by the general formula (a-3): 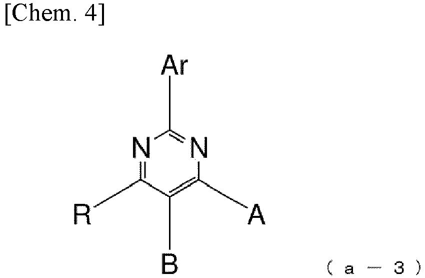 where A, B, Ar, and R are the same as defined in the general formula (a-1).
4) The present invention also provides the compound having a pyrimidine ring structure as set forth in clause 2), wherein A and B are different from each other.
5) The present invention also provides the compound having a pyrimidine ring structure as set forth in clause 3), wherein A and B are different from each other.
6) The present invention also provides the compound having a pyrimidine ring structure as set forth in clause 4), wherein L in the structural formula (b-1) represents a single bond, a substituted or unsubstituted phenyl group, a substituted or unsubstituted naphthyl group, a substituted or unsubstituted anthranil group, a substituted or unsubstituted phenanthrenyl group, or a substituted or unsubstituted biphenyl group.
7) The present invention also provides the compound having a pyrimidine ring structure as set forth in clause 5), wherein L in the structural formula (b-1) represents a single bond, a substituted or unsubstituted phenyl group, a substituted or unsubstituted naphthyl group, a substituted or unsubstituted anthranil group, a substituted or unsubstituted phenanthrenyl group, or a substituted or unsubstituted biphenyl group.
8) The present invention also provides the compound having a pyrimidine ring structure as set forth in clause 6), wherein Het in the structural formula (b-1) represents an unsubstituted pyridyl group, an unsubstituted quinolyl group, an unsubstituted isoquinolyl group, or an unsubstituted phenanthrolinyl group.
9) The present invention also provides the compound having a pyrimidine ring structure as set forth in clause 7), wherein Het in the structural formula (b-1) represents an unsubstituted pyridyl group, an unsubstituted quinolyl group, an unsubstituted isoquinolyl group, or an unsubstituted phenanthrolinyl group.
10) The present invention also provides an organic EL element including a pair of electrodes and one or more organic layers sandwiched therebetween, wherein the compound having a pyrimidine ring structure as set forth in any one of clauses 1) to 9) is included in at least one of the organic layers as a constituent material thereof.
11) The present invention also provides the organic EL element as set forth in clause 10), wherein the organic layer including the compound having a pyrimidine ring structure is an electron-transporting layer.
12) The present invention also provides the organic EL element as set forth in clause 10), wherein the organic layer including the compound having a pyrimidine ring structure is a hole-blocking layer.
13) The present invention also provides the organic EL element as set forth in clause 10), wherein the organic layer including the compound having a pyrimidine ring structure is a light-emitting layer.
14) The present invention also provides the organic EL element as set forth in clause 10), wherein the organic layer including the compound having a pyrimidine ring structure is used is an electron-injecting layer.

### Brief Description of Drawings

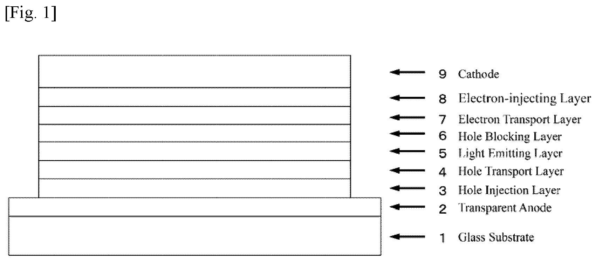

[Fig. 1] Fig. 1 is a diagram showing the configuration of organic EL elements of Examples 17 to 30 and Comparative Examples 1 to 4.

### Description of Embodiments

A compound having a pyrimidine ring structure of the present invention is a compound represented by the general formula (a-1) below: 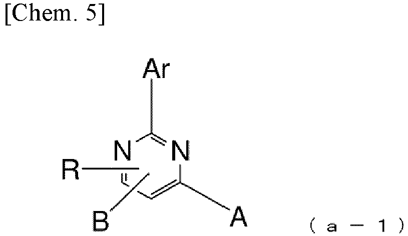 where A and B are the same or different from each other, and each are represented by the structural formula (b-1) below; Ar represents a substituted or unsubstituted aromatic hydrocarbon group, a substituted or unsubstituted aromatic heterocyclic group, or a substituted or unsubstituted fused polycyclic aromatic group; and R represents a hydrogen atom, a deuterium atom, a fluorine atom, a chlorine atom, a cyano group, a nitro group, a trimethylsilyl group, a triphenylsilyl group, a substituted or unsubstituted aromatic hydrocarbon group, a substituted or unsubstituted aromatic heterocyclic group, a substituted or unsubstituted fused polycyclic aromatic group, a linear or branched alkyl group having 1 to 6 carbon atoms and optionally having a substituent, a cycloalkyl group having 5 to 10 carbon atoms and optionally having a substituent, a linear or branched alkenyl group having 2 to 6 carbon atoms and optionally having a substituent, a linear or branched alkyloxy group having 1 to 6 carbon atoms and optionally having a substituent, or a cycloalkyloxy group having 5 to 10 carbon atoms and optionally having a substituent,
[Chem. 6]

**- - - - L-Het** (b - 1)

where L represents a single bond, or a divalent group of a substituted or unsubstituted aromatic hydrocarbon group, a substituted or unsubstituted aromatic heterocyclic group, or a substituted or unsubstituted fused polycyclic aromatic group; Het represents a substituted or unsubstituted pyridyl group, a substituted or unsubstituted pyrimidyl group, a substituted or unsubstituted triazyl group, a substituted or unsubstituted quinolyl group, a substituted or unsubstituted isoquinolyl group, a substituted or unsubstituted phenanthrolinyl group, a substituted or unsubstituted indolyl group, a substituted or unsubstituted azafluorenyl group, a substituted or unsubstituted diazafluorenyl group, a substituted or unsubstituted azaspirobifluorenyl group, or a substituted or unsubstituted diazaspirobifluorenyl group; and the dashed line represents a binding site to the pyrimidine ring.

Specific examples of the "aromatic hydrocarbon group", the "aromatic heterocyclic group", and the "fused polycyclic aromatic group" of the "substituted or unsubstituted aromatic hydrocarbon group", the "substituted or unsubstituted aromatic heterocyclic group", and the "substituted or unsubstituted fused polycyclic aromatic group" represented by Ar or R in the general formula (a-1) include a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzooxazolyl group, a benzothiazolyl group, an azafluorenyl group, a diazafluorenyl group, an azaspirobifluorenyl group, a diazaspirobifluorenyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, and a carbolinyl group; and also an aryl group having 6 to 30 carbon atoms, and a heteroaryl group having 2 to 20 carbon atoms.

Specific examples of the "substituent" of the "substituted aromatic hydrocarbon group", the "substituted aromatic heterocyclic group", and the "substituted fused polycyclic aromatic group" represented by Ar or R in the general formula (a-1) include a deuterium atom, a cyano group, and a nitro group; halogen atoms such as a fluorine atom, a chlorine atom, a bromine atom, and an iodine atom; silyl groups such as a trimethylsilyl group and a triphenylsilyl group; linear or branched alkyl groups having 1 to 6 carbon atoms such as a methyl group, an ethyl group, and a propyl group; linear or branched alkyloxy groups having 1 to 6 carbon atoms such as a methyloxy group, an ethyloxy group, and a propyloxy group; alkenyl groups such as a vinyl group and an allyl group; aryloxy groups such as a phenyloxy group and a tolyloxy group; arylalkyloxy groups such as a benzyloxy group and a phenethyloxy group; aromatic hydrocarbon groups or fused polycyclic aromatic groups such as a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, and a triphenylenyl group; and aromatic heterocyclic groups such as a pyridyl group, a thienyl group, a furyl group, a pyrrolyl group, a quinolyl group, an isoquinolyl group, a benzofuranyl group, a benzothienyl group, an indolyl group, a carbazolyl group, a benzooxazolyl group, a benzothiazolyl group, a quinoxalinyl group, a benzimidazolyl group, a pyrazolyl group, a dibenzofuranyl group, a dibenzothienyl group, and a carbolinyl group. These substituents may further be substituted by any of the substituents listed above as examples. Moreover, a substituted benzene ring and the substituent on the benzene ring, or a plurality of substituents that substitute the same benzene ring may be bonded to each other to form a ring via a single bond, a substituted or unsubstituted methylene group, an oxygen atom, or a sulfur atom.

Specific examples of the "linear or branched alkyl group having 1 to 6 carbon atoms", the "cycloalkyl group having 5 to 10 carbon atoms", and the "linear or branched alkenyl group having 2 to 6 carbon atoms" of the "linear or branched alkyl group having 1 to 6 carbon atoms and optionally having a substituent", the "cycloalkyl group having 5 to 10 carbon atoms and optionally having a substituent", and the "linear or branched alkyl group having 1 to 6 carbon atoms" represented by R in the general formula (a-1) include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, and a 2-butenyl group. A substituted benzene ring and the substituent on the benzene ring, or a plurality of substituents that substitute the same benzene ring may be bonded to each other to form a ring via a single bond, a substituted or unsubstituted methylene group, a substituted or unsubstituted amino group, an oxygen atom, or a sulfur atom.

Examples of the "substituent" of the "linear or branched alkyl group having 1 to 6 carbon atoms and optionally having a substituent", the "cycloalkyl group having 5 to 10 carbon atoms and optionally having a substituent", and the "linear or branched alkenyl group having 2 to 6 carbon atoms and optionally having a substituent" represented by R in the general formula (a-1) are the same as those listed above as examples of the "substituent" of the "substituted aromatic hydrocarbon group", the "substituted aromatic heterocyclic group", and the "substituted fused polycyclic aromatic group" represented by Ar or R in the general formula (a-1). The same holds true for the form that the substituent may be in.

Specific examples of the "linear or branched alkyloxy group having 1 to 6 carbon atoms" and the "cycloalkyloxy group having 5 to 10 carbon atoms" of the "linear or branched alkyloxy group having 1 to 6 carbon atoms and optionally having a substituent" and the "cycloalkyloxy group having 5 to 10 carbon atoms and optionally having a substituent" represented by R in the general formula (a-1) include a methyloxy group, an ethyloxy group, an n-propyloxy group, an isopropyloxy group, an n-butyloxy group, a tert-butyloxy group, an n-pentyloxy group, an n-hexyloxy group, a cyclopentyloxy group, a cyclohexyloxy group, a cycloheptyloxy group, a cyclooctyloxy group, a 1-adamantyloxy group, and a 2-adamantyloxy group. A substituted benzene ring and the substituent on the benzene ring, or a plurality of substituents that substitute the same benzene ring may be bonded to each other to form a ring via a single bond, a substituted or unsubstituted methylene group, a substituted or unsubstituted amino group, an oxygen atom, or a sulfur atom.

Examples of the "substituent" of the "linear or branched alkyloxy group having 1 to 6 carbon atoms and optionally having a substituent" and the "cycloalkyloxy group having 5 to 10 carbon atoms and optionally having a substituent" represented by R in the general formula (a-1) are the same as those listed above as examples of the "substituent" of the "substituted aromatic hydrocarbon group", the "substituted aromatic heterocyclic group", and the "substituted fused polycyclic aromatic group" represented by Ar or R in the general formula (a-1). The same holds true for the form that the substituent may be in.

Examples of the "aromatic hydrocarbon group", the "aromatic heterocyclic group", and the "fused polycyclic aromatic group" of the "substituted or unsubstituted aromatic hydrocarbon group", the "substituted or unsubstituted aromatic heterocyclic group", and the "substituted or unsubstituted fused polycyclic aromatic group" represented by L in the structural formula (b-1) include divalent groups obtained by removing one hydrogen atom from the groups listed above as examples of the "aromatic hydrocarbon group", the "aromatic heterocyclic group", and the "fused polycyclic aromatic group" of the "substituted or unsubstituted aromatic hydrocarbon group", the "substituted or unsubstituted aromatic heterocyclic group", and the "substituted or unsubstituted fused polycyclic aromatic group" represented by Ar or R in the general formula (a-1). The same holds true for the form that the group may be in.

Examples of the "substituent" of the "substituted aromatic hydrocarbon group", the "substituted aromatic heterocyclic group", and the "substituted fused polycyclic aromatic group" represented by L in the structural formula (b-1) are the same as those listed above as examples of the "substituent" of the "substituted aromatic hydrocarbon group", the "substituted aromatic heterocyclic group", and the "substituted fused polycyclic aromatic group" represented by Ar or R in the general formula (a-1). The same holds true for the form that the substituent may be in.

Examples of the "substituent" of the "substituted pyridyl group", the "substituted pyrimidyl group", the "substituted triazyl group", the "substituted quinolyl group", the "substituted isoquinolyl group", the "substituted phenanthrolinyl group", the "substituted indolyl group", the "substituted azafluorenyl group", the "substituted diazafluorenyl group", the "substituted azaspirobifluorenyl group", and the "substituted diazaspirobifluorenyl group" represented by Het in the structural formula (b-1) are the same as those listed above as examples of the "substituent" of the "substituted aromatic hydrocarbon group", the "substituted aromatic heterocyclic group", and the "substituted fused polycyclic aromatic group" represented by Ar or R in the general formula (a-1). The same holds true for the form that the substituent may be in.

In the present invention, in view of electron-injecting/transporting capability, hole-blocking capability, stability in the form of a thin film, etc., Ar in the general formula (a-1) is preferably a substituted or unsubstituted aromatic hydrocarbon group or a substituted or unsubstituted fused polycyclic aromatic group, and more preferably a substituted or unsubstituted phenyl group, a substituted or unsubstituted anthracenyl group, a substituted or unsubstituted phenanthrenyl group, a substituted or unsubstituted fluorenyl group, or a substituted or unsubstituted spirobifluorenyl group.

R is preferably a hydrogen atom.

In the present invention, in view of electron-injecting/transporting capability, hole-blocking capability, stability in the form of a thin film, etc., L in the structural formula (b-1) is preferably a single bond, a substituted or unsubstituted phenylene group, a substituted or unsubstituted naphthylene group, a substituted or unsubstituted anthranylene group, a substituted or unsubstituted phenanthrenylene group, or a substituted or unsubstituted biphenylene group, and more preferably an unsubstituted phenylene group.

Het is preferably an unsubstituted pyridyl group, an unsubstituted quinolyl group, or an unsubstituted isoquinolyl group.

In view of electron-injecting/transporting capability, hole-blocking capability, stability in the form of a thin film, etc., the compound having a pyrimidine ring structure of the present invention is preferably a compound represented by the general formula (a-2) or (a-3) below. 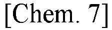 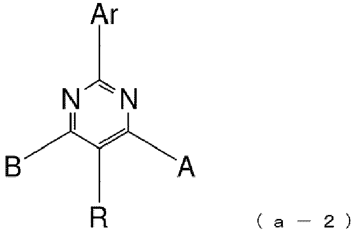 where A, B, Ar, and R are the same as defined in the general formula (a-1). 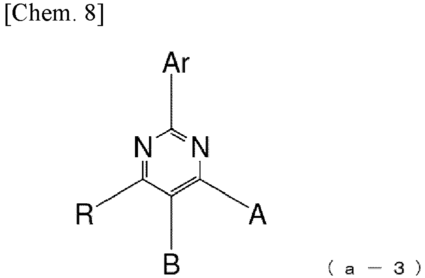 where A, B, Ar, and R are the same as defined in the general formula (a-1).

In view of electron-injecting/transporting capability, hole-blocking capability, stability in the form of a thin film, etc., the compound having a pyrimidine ring structure of the present invention is more preferably represented by the general formula (a-2).

That is to say, the compound having a pyrimidine ring structure of the present invention is most preferably represented by the general formula (a-2) with the following conditions fulfilled: Ar is a substituted or unsubstituted phenyl group, a substituted or unsubstituted anthracenyl group, a substituted or unsubstituted phenanthrenyl group, a substituted or unsubstituted fluorenyl group, or a substituted or unsubstituted spirobifluorenyl group, R is a hydrogen atom, L is an unsubstituted phenyl group, and Het is an unsubstituted pyridyl group, an unsubstituted quinolyl group, or an unsubstituted isoquinolyl group.

The compound having a pyrimidine ring structure of the present invention has the properties including: (1) good electron-injecting properties, (2) high electron mobility, (3) excellent hole-blocking capability, (4) stability in the form of a thin film, and (5) excellent heat resistance. The organic EL element of the present invention has the characteristics including: (1) high luminous efficacy and high power efficiency, (2) a low voltage for the start of light emission, (3) a low driving voltage in actual use, and (4) a long lifespan.

The compound having a pyrimidine ring structure of the present invention has good electron-injecting properties and high electron mobility. Therefore, an organic EL element having an electron-injecting layer and/or an electron-transporting layer including the compound as an electron-injecting material and/or an electron-transporting material has improved efficiency in terms of transporting electrons to the light-emitting layer and hence improved luminous efficacy, and also has a lower driving voltage and hence improved durability.

The compound having a pyrimidine ring structure of the present invention is characterized in excellent hole-blocking capability and electron transportability, stability in the form of a thin film, and capability to confine excitons generated in a light-emitting layer. Therefore, an organic EL element including a hole-blocking layer prepared by using the compound as a hole-blocking material has high luminous efficacy because the probability of recombination of holes and electrons is increased to thereby suppress the heat inactivation, and also has an increased maximum luminance because the driving voltage is reduced to thereby improve the current resistance.

The compound having a pyrimidine ring structure of the present invention has excellent electron-transporting capability and has a wide band gap. Therefore, an organic EL element including a light-emitting layer prepared by using the compound as a host material has a reduced driving voltage and hence improved luminous efficacy when the light-emitting layer contains a fluorescent emitter, a phosphorescent emitter, or a delayed fluorescent emitter, which are called dopants.

Accordingly, the compound having a pyrimidine ring structure of the present invention is useful as the material of an electron-injecting layer, an electron-transporting layer, a hole-blocking layer, or a light-emitting layer of an organic EL element, and can improve the luminous efficacy, the driving voltage, and the durability of a conventional organic EL element.

Although the compound having a pyrimidine ring structure of the present invention is a novel compound, the compound itself can be synthesized according to a known method (see Patent Literature 5 and Non-Patent Literature 6, for example).

The compound having a pyrimidine ring structure of the present invention can be purified by, for example, column chromatography, adsorption with silica gel, activated carbon, activated clay, or others, recrystallization or crystallization from a solvent, or sublimation.

Preferred examples of the compound of the present invention having a pyrimidine ring structure and represented by the general formula (a-1) will specifically be given below. However, the present invention is not limited to these compounds.

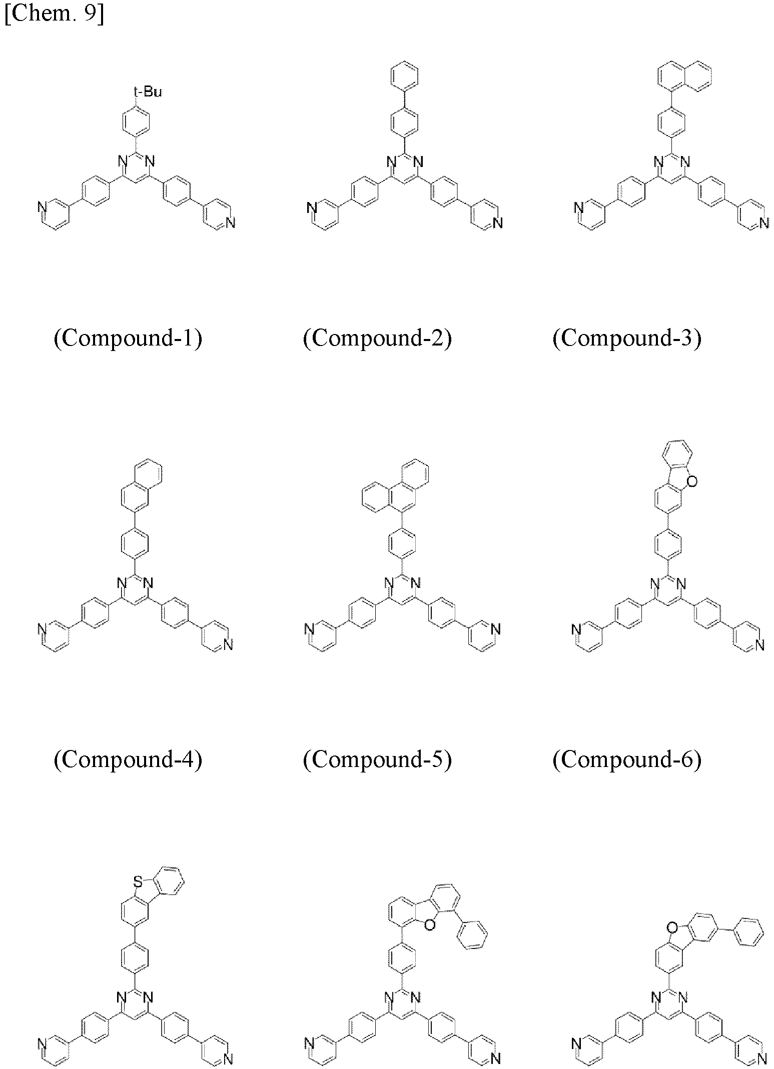

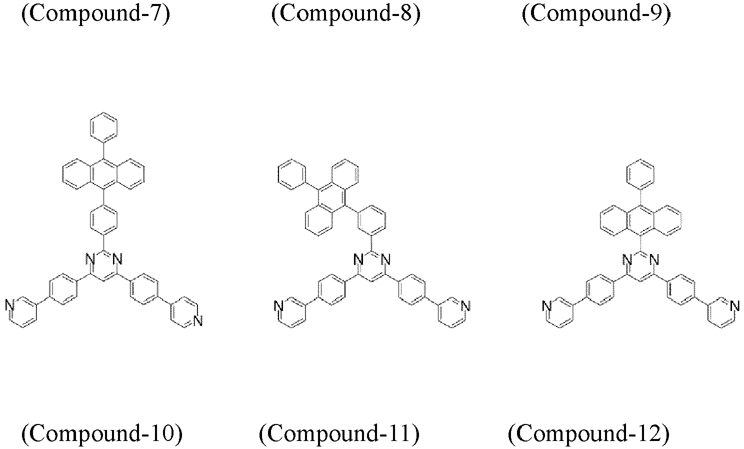

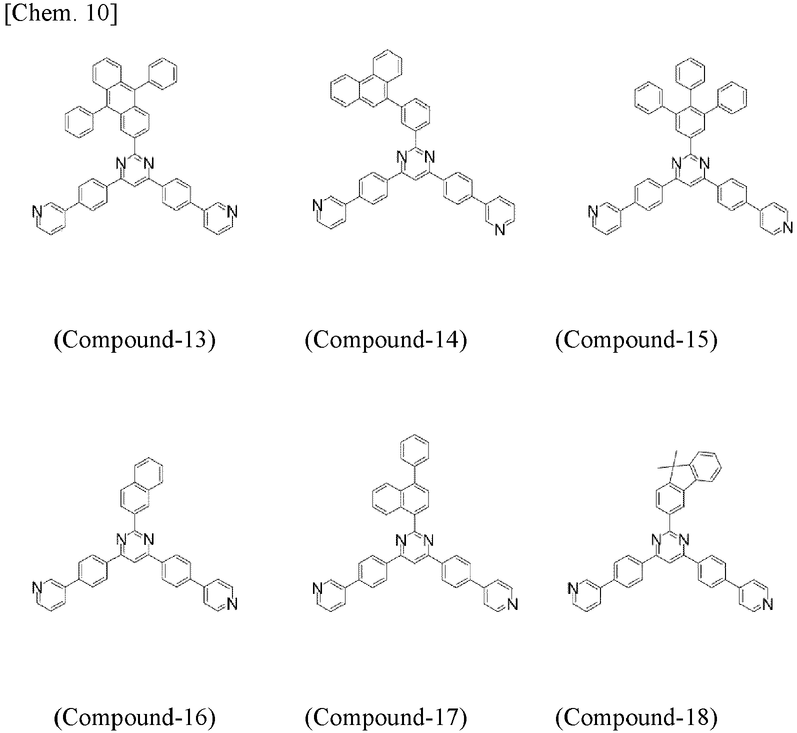

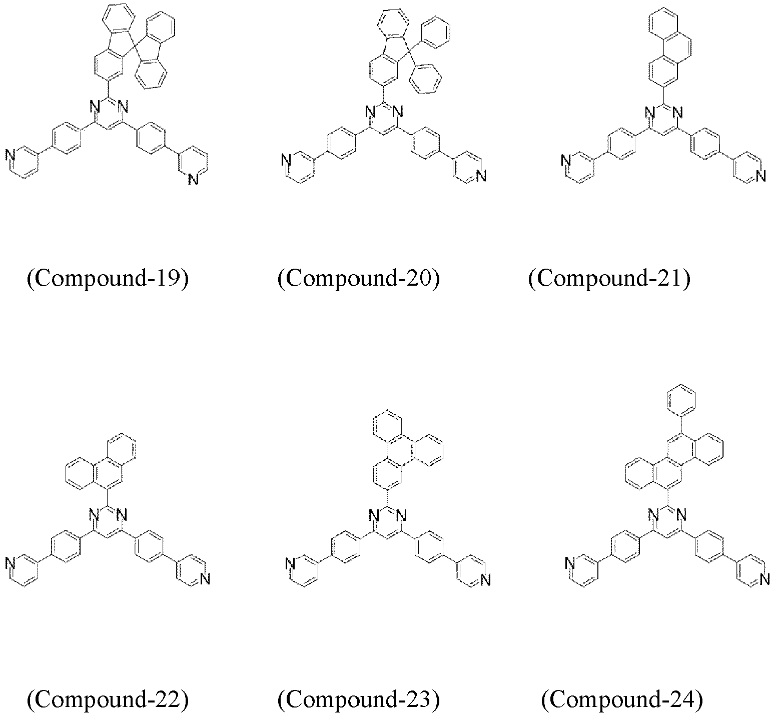

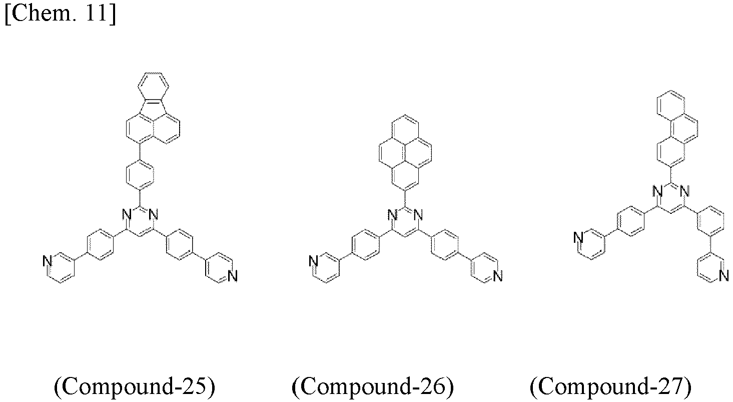

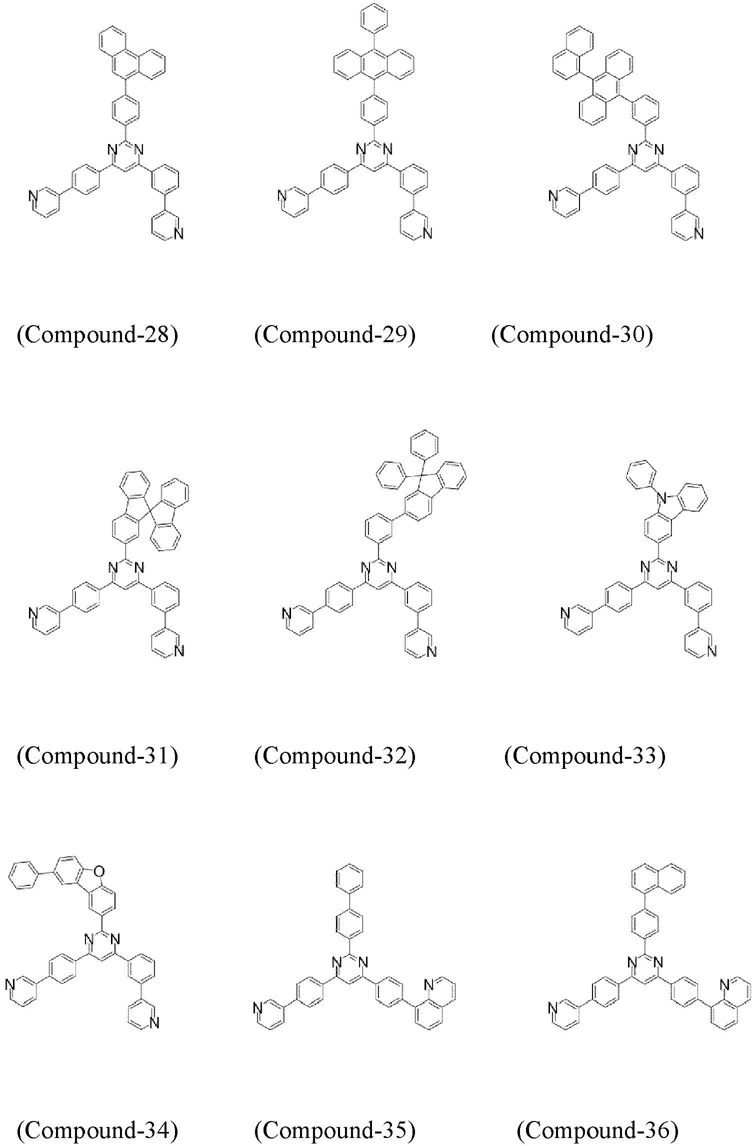

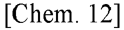

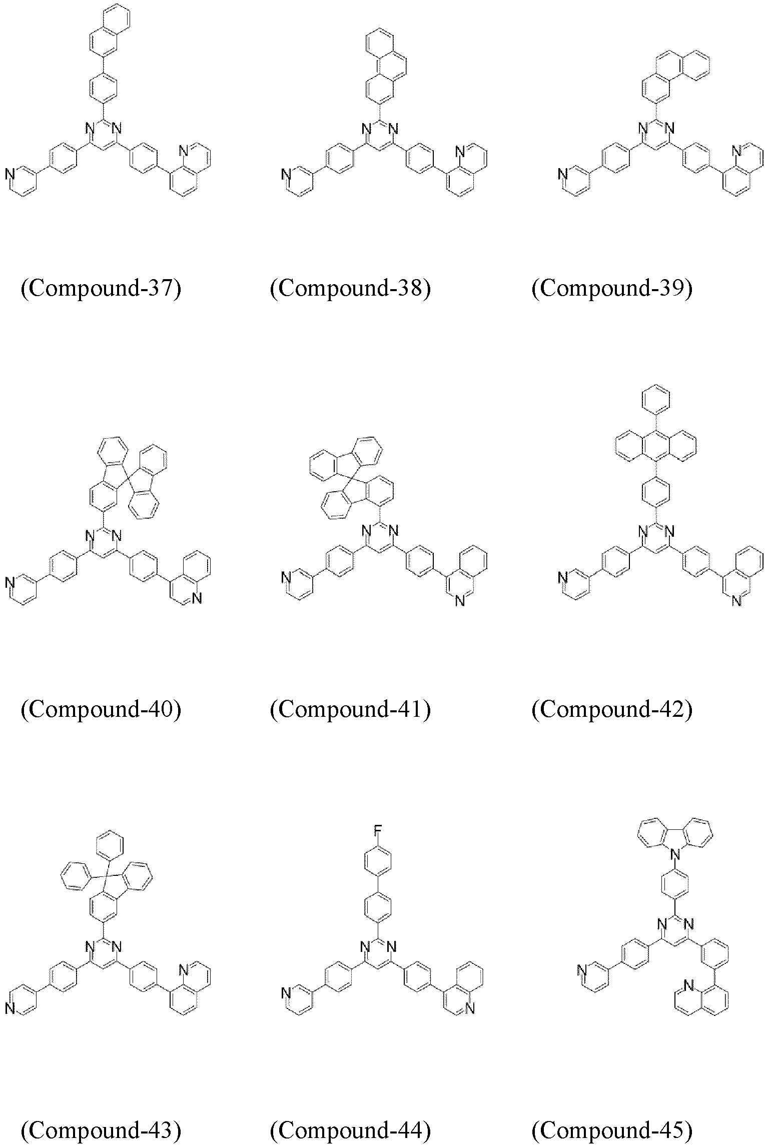

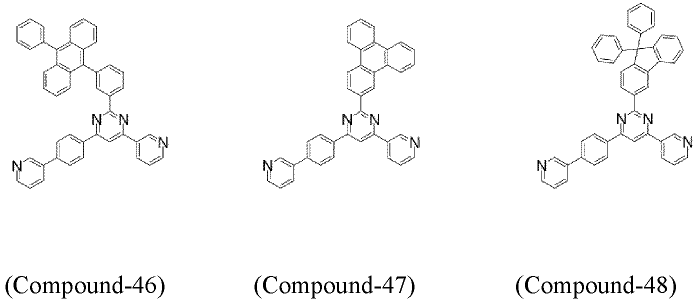

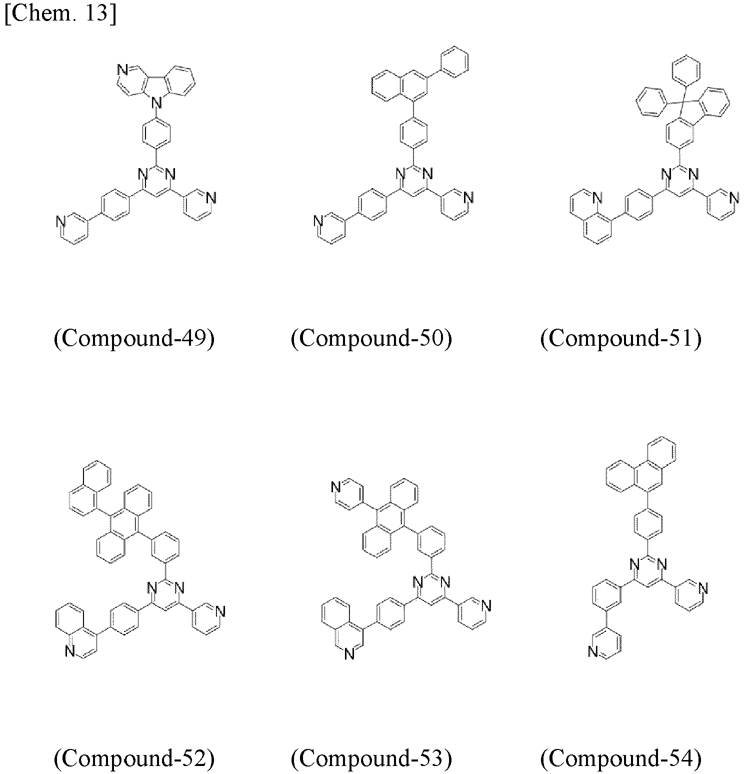

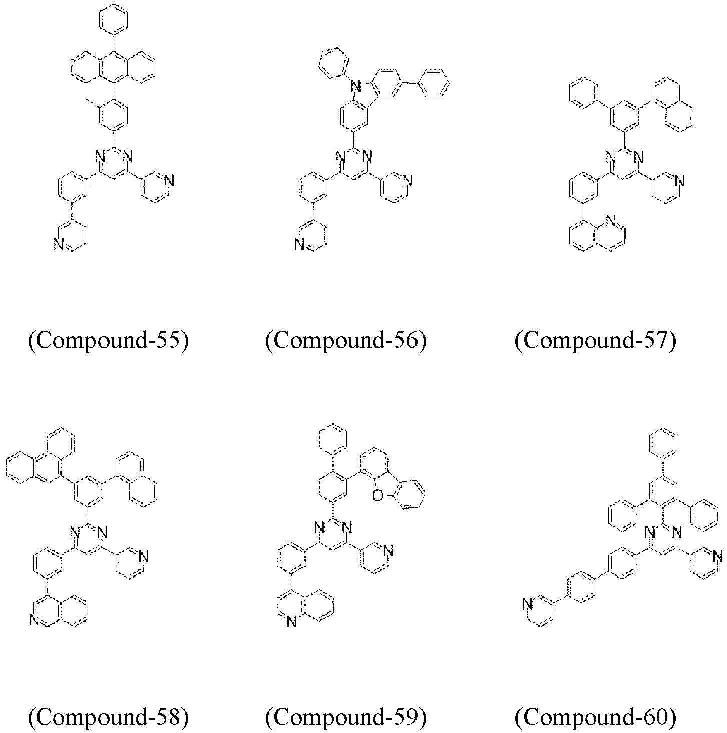

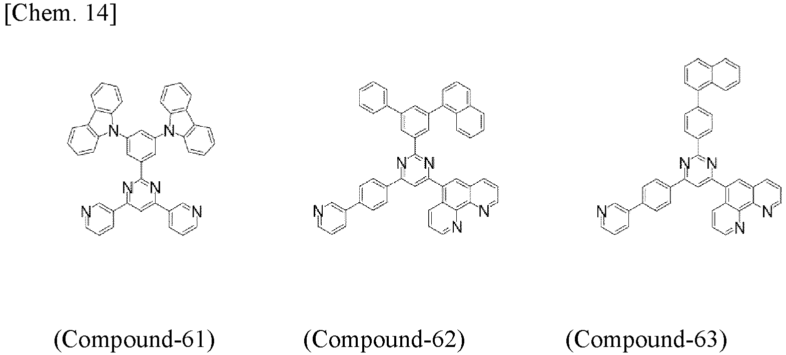

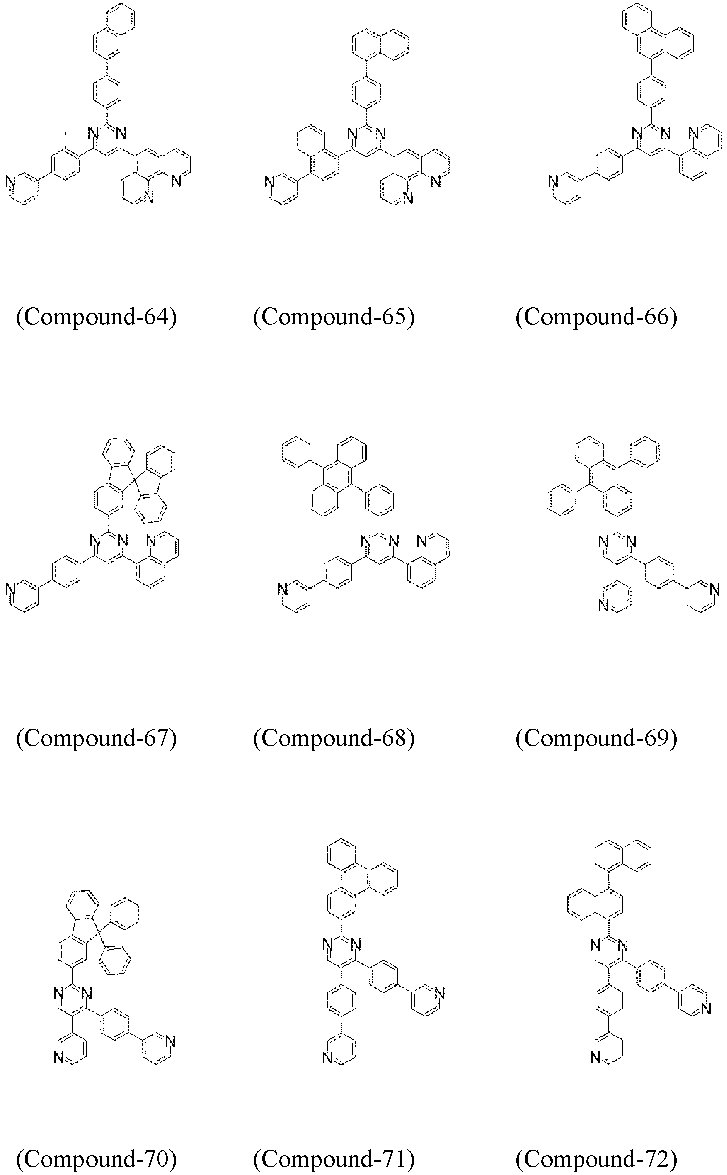

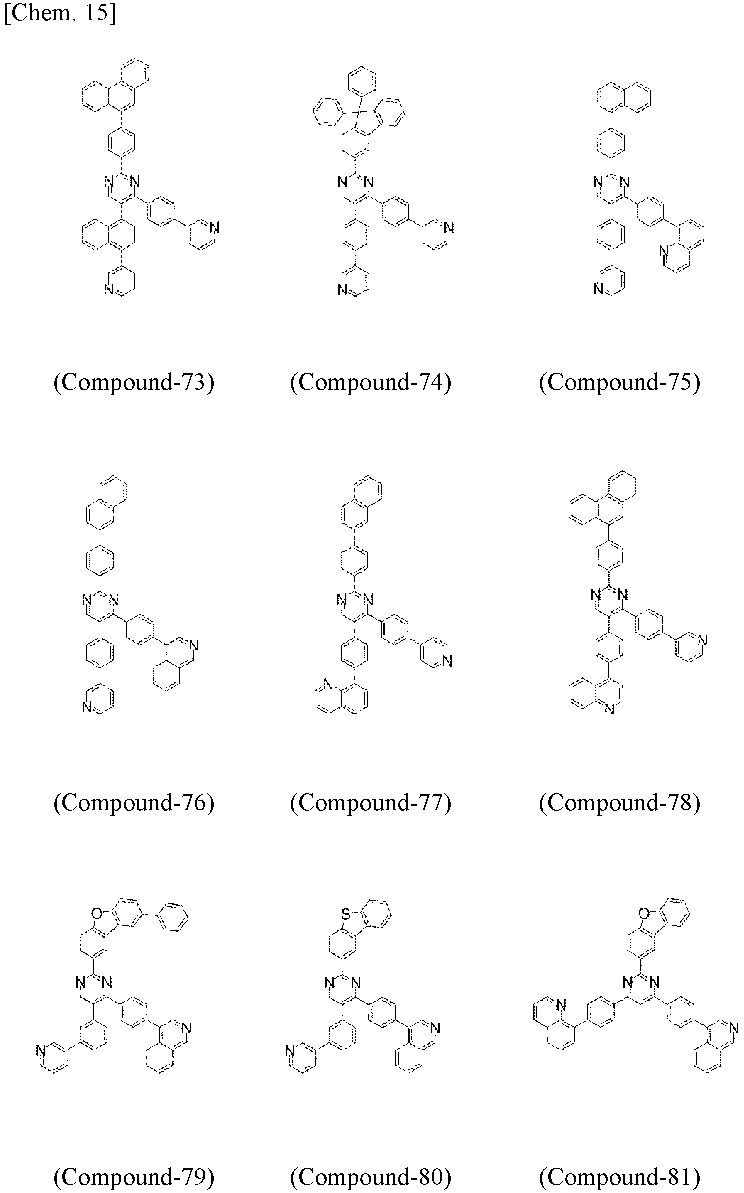

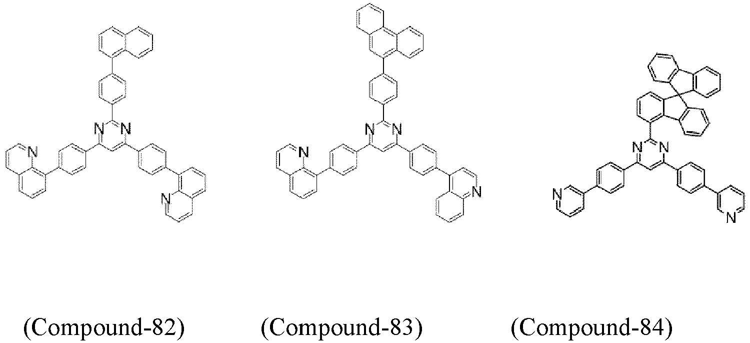

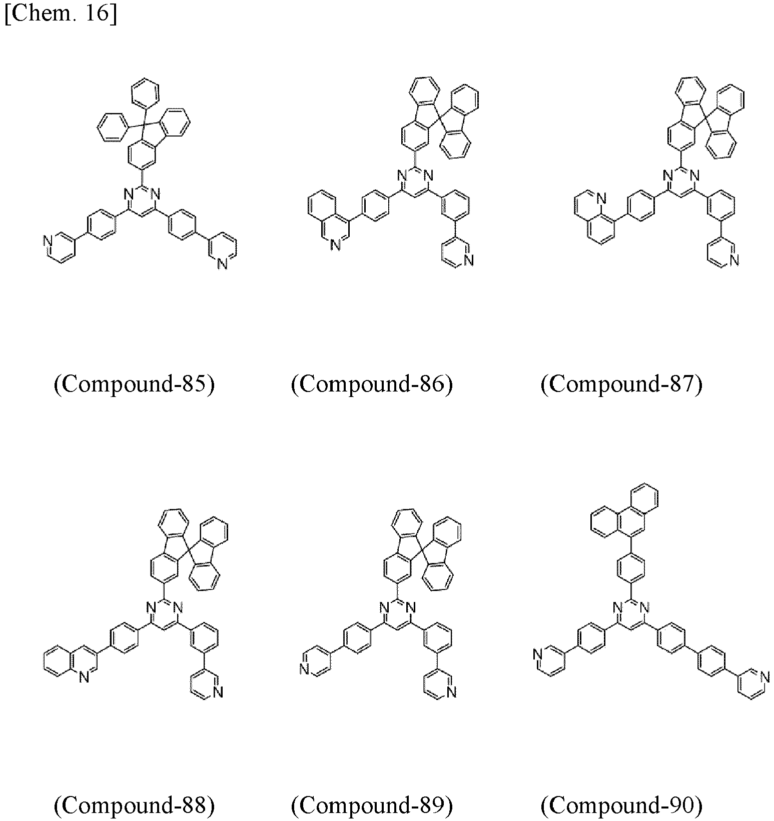

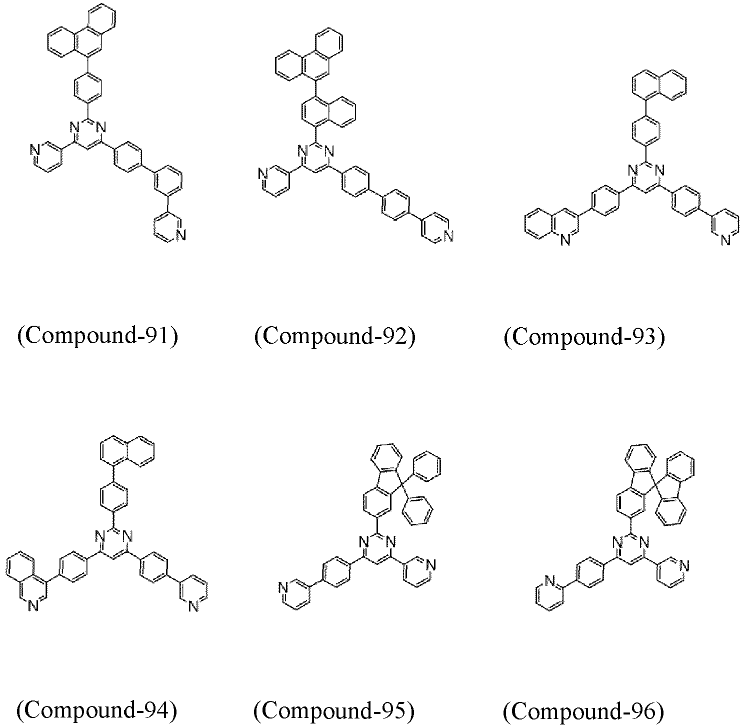

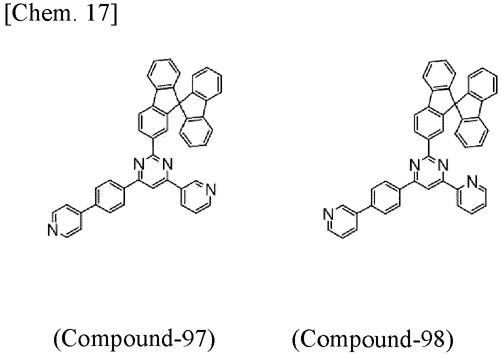

The compound having a pyrimidine ring structure of the present invention can be used as a constituent material of a light-emitting layer, an electron-injecting layer, an electron-transporting layer, and a hole-blocking layer of an organic EL element. In particular, the compound has high electron mobility, and is therefore suitable as a constituent material of an electron-injecting layer or an electron-transporting layer.

An organic EL element of the present invention has a pair of electrodes and one or more organic layers sandwiched therebetween, wherein the compound having a pyrimidine ring structure of the present invention is included in at least one of the organic layers as a constituent material thereof. The organic layers may be a hole-injecting layer, a hole-transporting layer, an electron-blocking layer, a light-emitting layer, a hole-blocking layer, an electron-transporting layer, an electron-injecting layer, and the like, and the compound having a pyrimidine ring structure of the present invention can be suitably used as a constituent material of the light-emitting layer, the hole-blocking layer, the electron-transporting layer, and the electron-injecting layer.

The organic EL element of the present invention may have a structure in which an anode, a hole-injecting layer, a hole-transporting layer, a light-emitting layer, an electron-transporting layer, an electron-injecting layer, and a cathode are sequentially provided on a substrate; and the structure may further include an electron-blocking layer between the hole-transporting layer and the light-emitting layer and also may further include a hole-blocking layer between the light-emitting layer and the electron-transporting layer. In these multilayer structures, one or more organic layers may be omitted. Also, a single layer may serve as both the hole-injecting layer and the hole-transporting layer, and a single layer may serve as both the electron-injecting layer and the electron-transporting layer, for example. Moreover, it is possible to stack two or more organic layers having the same function. Specifically, two hole-transporting layers may be stacked; two light-emitting layers may be stacked; and two electron-transporting layers may be stacked.

An electrode material having a high work function, such as ITO or gold, is used for the anode of the organic EL element of the present invention. Examples of a material used for the hole-injecting layer of the organic EL element of the present invention include porphyrin compounds typified by copper phthalocyanine; starburst triphenylamine derivatives; arylamine compounds having a structure containing two or more triphenylamine structures or carbazolyl structures in the molecule, the triphenylamine or the carbazolyl structures being linked via a single bond or a divalent group having no heteroatom; heterocyclic compounds of acceptor type, such as hexacyanoazatriphenylene; polymer materials of coating type. These materials can be formed into a thin film using a known method such as vapor deposition, spin coating, or inkjet printing.

Examples of a material used for the hole-transporting layer of the organic EL element of the present invention include benzidine derivatives, such as N,N'-diphenyl-N,N'-di(m-tolyl)-benzidine (hereinafter abbreviated as TPD), N,N'-diphenyl-N,N'-di(α-naphthyl)benzidine (hereinafter abbreviated as NPD), and N,N,N',N'-tetrabiphenylyl benzidine; 1,1-bis[(di-4-tolylamino)phenyl]cyclohexane (hereinafter abbreviated as TAPC); and arylamine compounds having a structure containing two or more triphenylamine structures or carbazolyl structures in the molecule, the triphenylamine or the carbazolyl structures being linked via a single bond or a divalent group having no heteroatom. These materials may be used singly for film formation, or a mixture of any of these material and another material may be used to form a single layer. The hole-transporting layer may have a layered structure composed of different layers each formed of a single kind of the materials described above, a layered structure composed of layers each formed of a mixture of two or more of the materials described above, or a layered structure composed of a layer formed of a single kind of the materials described above and a layer formed of a mixture of two or more of the materials described above. It is also possible to use polymer materials of coating type, such as poly(3,4-ethylenedioxythiophene) (hereinafter abbreviated as PEDOT)/poly(styrenesulfonate) (hereinafter abbreviated as PSS), for a hole injecting/transporting layer. These materials can be formed into a thin film using a known method such as vapor deposition, spin coating, or inkjet printing.

Other examples of the material used for the hole-injecting layer or the hole-transporting layer include a material obtained by p-doping a material normally used for the hole-injecting layer or the hole-transporting layer with trisbromophenylamine hexachloroantimony or a radialene derivative (see Patent Literature 7, for example); and a polymer compound having the structure of a benzidine derivative, such as TPD, as a partial structure thereof.

Examples of a material used for the electron-blocking layer of the organic EL element of the present invention include compounds having an electron-blocking effect, such as carbazole derivatives such as 4,4',4"-tri(N-carbazolyl)triphenylamine (hereinafter abbreviated as TCTA), 9,9-bis[4-(carbazole-9-yl)phenyl]fluorene, 1,3-bis(carbazole-9-yl)benzene (hereinafter abbreviated as mCP), and 2,2-bis(4-carbazole-9-ylphenyl)adamantane (hereinafter abbreviated as Ad-Cz); and compounds having a triphenylsilyl group and a triarylamine structure and typified by 9-[4-(carbazole-9-yl)phenyl]-9-[4-(triphenylsilyl)phenyl]-9H-fluorene . These materials may be used singly for film formation, or a mixture of any of these material and another material may be used to form a single layer. The electron-blocking layer may have a layered structure composed of different layers each formed of a single kind of the materials described above, a layered structure composed of layers each formed of a mixture of two or more of the materials described above, or a layered structure composed of a layer formed of a single kind of the materials described above and a layer formed of a mixture of two or more of the materials described above. These materials can be formed into a thin film using a known method such as vapor deposition, spin coating, or inkjet printing.

Examples of a material used for the light-emitting layer of the organic EL element of the present invention include the compound having a pyrimidine ring structure of the present invention, and also metal complexes of quinolinol derivatives such as Alq₃, various types of metal complexes, an anthracene derivative, a bisstyrylbenzene derivative, a pyrene derivative, an oxazole derivative, and a polyphenylene vinylene derivative. The light-emitting layer may include a host material and a dopant material. As the host material, an anthracene derivative is preferably used. Other examples of the host material include the above-listed light-emitting materials including the compound having a pyrimidine ring structure of the present invention, and also heterocyclic compound having an indole ring as a partial structure of a fused ring, a heterocyclic compound having a carbazole ring as a partial structure of a fused ring, a carbazole derivative, a thiazole derivative, a benzimidazole derivative, and a polydialkylfluorene derivative. Examples of the dopant material include quinacridone, coumalin, rubrene, perylene, and derivatives thereof; a benzopyran derivative; a rhodamine derivative; and an aminostyryl derivative. These materials may be used singly for film formation, or a mixture of any of these material and another material may be used to form a single layer. The light-emitting layer may have a layered structure composed of different layers each formed of a single kind of the materials described above, a layered structure composed of layers each formed of a mixture of two or more of the materials described above, or a layered structure composed of a layer formed of a single kind of the materials described above and a layer formed of a mixture of two or more of the materials described above. These materials can be formed into a thin film using a known method such as vapor deposition, spin coating, or inkjet printing.

A phosphorescent emitter can also be used as a light-emitting material. The phosphorescent emitter may be a metal complex of iridium, platinum, or the like, and examples thereof include a green phosphorescent emitter such as Ir(ppy)₃, a blue phosphorescent emitter such as FIrpic or FIr6, and a red phosphorescent emitter such as Btp₂Ir (acac). As a host material in this case, a host material having hole-injecting/transporting capability may be used, including carbazole derivatives such as 4,4'-di(N-carbazolyl)biphenyl (hereinafter abbreviated as CBP), TCTA, and mCP, and also the compound having a pyrimidine ring structure of the present invention. Also, a host material having electron-transporting capability may be used, including p-bis(triphenylsilyl)benzene (hereinafter abbreviated as UGH2) and 2,2',2"-(1,3,5-phenylene)-tris(1-phenyl-1H-benzimidazole) (hereinafter abbreviated as TPBI), and a high-performance organic EL element can thus be produced.

In order to avoid concentration quenching, doping of the host material with a phosphorescent material is preferably performed by co-deposition in an amount within a range of 1 to 30 wt% based on the entire light-emitting layer.

As the light-emitting material, a material that emits delayed fluorescence can also be used, including a CDCB derivative, such as PIC-TRZ, CC2TA, PXZ-TRZ, or 4CzIPN (see Non-Patent Literature 3, for example). These materials can be formed into a thin film using a known method such as vapor deposition, spin coating, or inkjet printing.

Examples of a material used for the hole-blocking layer of the organic EL element of the present invention include the compound having a pyrimidine ring structure of the present invention, and also compounds having a hole-blocking effect, including a phenanthroline derivative, such as bathocuproine (hereinafter abbreviated as BCP); a metal complex of a quinolinol derivative, such as BAlq; various types of rare-earth complexes; an oxazole derivative; a triazole derivative; and a triazine derivative. These materials may also serve as the material of the electron-transporting layer. These materials may be used singly for film formation, or a mixture of any of these material and another material may be used to form a single layer. The hole-blocking layer may have a layered structure composed of different layers each formed of a single kind of the materials described above, a layered structure composed of layers each formed of a mixture of two or more of the materials described above, or a layered structure composed of a layer formed of a single kind of the materials described above and a layer formed of a mixture of two or more of the materials described above. These materials can be formed into a thin film using a known method such as vapor deposition, spin coating, or inkjet printing.

Examples of a material used for the electron-transporting layer of the organic EL element of the present invention include the compound having a pyrimidine ring structure of the present invention, and also metal complexes of quinolinol derivatives, such as Alq₃ and BAlq; various types of metal complexes; a triazole derivative; a triazine derivative; an oxadiazole derivative; a pyridine derivative; a benzimidazole derivative; a thiadiazole derivative; an anthracene derivative; a carbodiimide derivative; a quinoxaline derivative; a pyridoindole derivative; a phenanthroline derivative; and a silole derivative. These materials may be used singly for film formation, or a mixture of any of these material and another material may be used to form a single layer. The electron-transporting layer may have a layered structure composed of different layers each formed of a single kind of the materials described above, a layered structure composed of layers each formed of a mixture of two or more of the materials described above, or a layered structure composed of a layer formed of a single kind of the materials described above and a layer formed of a mixture of two or more of the materials described above. These materials can be formed into a thin film using a known method such as vapor deposition, spin coating, or inkjet printing.

Examples of a material used for the electron-injecting layer of the organic EL element of the present invention include the compound having a pyrimidine ring structure of the present invention, and also an alkali metal salt such as lithium fluoride or cesium fluoride; an alkaline earth metal salt such as magnesium fluoride; a metal complex of a quinolinol derivative such as lithium quinolinol; a metal oxide such as aluminum oxide; and a metal such as ytterbium (Yb), samarium (Sm), calcium (Ca), strontium (Sr), and cesium (Cs). The electron-injecting layer can however be omitted when an electron-transporting layer and a cathode are suitably selected.

Furthermore, a material obtained by n-doping a material normally used for the electron-injecting layer or the electron-transporting layer with a metal such as cesium can be used for the electron-injecting layer or the electron-transporting layer.

Examples of an electrode material used for the cathode of the organic EL element of the present invention include an electrode material having a low work function, such as aluminum; or an alloy having an even lower work function, such as a magnesium-silver alloy, a magnesium-indium alloy, or an aluminum-magnesium alloy.

The organic EL element of the present invention uses, as a constituent material, the compound having a pyrimidine ring structure of the present invention, which is excellent in electron-injecting/transporting capability, stability in the form of a thin film, and durability. Therefore, compared with a conventional organic EL element, the organic EL element of the present invention has improved efficiency in terms of transporting electrons from the electron-transporting layer to the light-emitting layer and hence improved luminous efficacy, as well as a reduced driving voltage and hence improved durability, and thus the organic EL element of the present invention can achieve high efficiency, a low driving voltage, and a long lifespan.

### Examples

Hereinafter, embodiments of the present invention will be described in greater detail by way of Examples. However, the present invention is not limited to Examples below as long as it does not depart from the gist thereof.

### Example 1

### <Synthesis of 2-{4-(phenanthrene-9-yl)-phenyl}-4,6-bis-{4-(pyridine-3-yl)-phenyl}-pyrimidine (Compound-5)>

First, 11.0 g of 4,6-bis-(4-chloro-phenyl)-2-{4-(phenanthrene-9-yl)-phenyl}-pyrimidine, 5.9 g of 3-pyridylboronic acid, 0.9 g of tris(dibenzylideneacetone)dipalladium(0), 1.1 g of tricyclohexylphosphine, and 8.2 g of potassium carbonate were placed in a reaction vessel, and stirred under reflux overnight in a 1,4-dioxane/H₂O mixed solvent. The reaction system was allowed to cool. Then, H₂O was added thereto, and the deposited solid was collected by filtering to thereby obtain a crude product. The obtained crude product was purified through recrystallization from a monochlorobenzene solvent to thereby obtain 7.9 g (yield: 62%) of a white powder of 2-{4-(phenanthrene-9-yl)-phenyl}-4,6-bis-{4-(pyridine-3-yl)-phenyl}-pyrimidine (Compound-5).

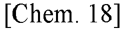

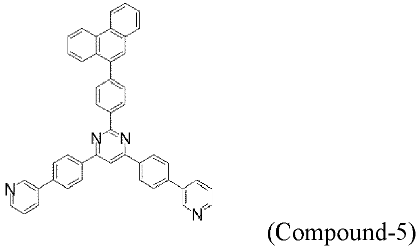

The structure of the obtained white powder was identified using NMR

In ¹H-NMR (CDCl₃), the following signals of 30 hydrogens were detected.
δ (ppm) = 9.00 (2H), 8.93 (2H), 8.84 (1H), 8.78 (1H), 8.69 (2H), 8.51 (4H), 8.18 (1H), 8.08-7.94 (4H), 7.88-7.55 (11H), 7.47 (1H), 7.45 (1H)

### Example 2

### <Synthesis of 2-(10-phenyl-anthracene-9-yl)-4,6-bis-{4-(pyridine-3-yl)-phenyl}-pyrimidine (Compound-12)>

First, 5.2 g of 4,6-bis-(4-chloro-phenyl)-2-(10-phenyl-anthracene-9-yl)-pyrimidine, 2.8 g of 3-pyridylboronic acid, 0.4 g of tris(dibenzylideneacetone)dipalladium(0), 0.5 g of tricyclohexylphosphine, and 3.9 g of potassium carbonate were placed in a reaction vessel, and stirred under reflux overnight in a 1,4-dioxane/H₂O mixed solvent. The reaction system was allowed to cool. Then, H₂O was added thereto, and the deposited solid was collected by filtering to thereby obtain a crude product. The obtained crude product was purified through recrystallization from a monochlorobenzene solvent to thereby obtain 1.5 g (yield: 25%) of a white powder of 2-(10-phenyl-anthracene-9-yl)-4,6-bis-{4-(pyridine-3-yl)-phenyl}-pyrimidine (Compound-12).

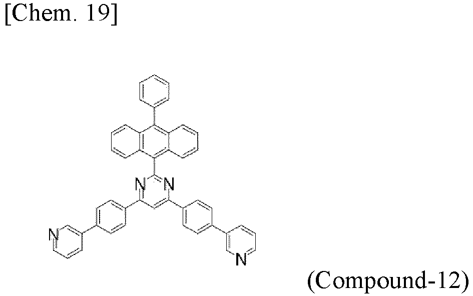

The structure of the obtained white powder was identified using NMR

In ¹H-NMR (CDCl₃), the following signals of 30 hydrogens were detected.
δ (ppm) = 8.95 (2H), 8.66 (2H), 8.44 (4H), 8.40 (1H), 7.98 (2H), 7.85 (2H), 7.79 (4H), 7.76 (2H), 7.69-7.57 (3H), 7.52 (2H), 7.48-7.34 (6H)

### Example 3

### <Synthesis of 4,6-bis-{4-(pyridine-3-yl)-phenyl}-2-(9,9'-spirobi[9H]fluorene-2-yl)-pyrimidine (Compound-19)>

First, 8.0 g of 4,6-bis-(4-chloro-phenyl)-2-(9,9'-spirobi[9H]fluorene-2-yl)-pyrimidine, 3.8 g of 3-pyridylboronic acid, 0.6 g of tris(dibenzylideneacetone)dipalladium(0), 0.7 g of tricyclohexylphosphine, and 5.4 g of potassium carbonate were placed in a reaction vessel, and stirred under reflux overnight in a 1,4-dioxane/H₂O mixed solvent. The reaction system was allowed to cool. Then, H₂O was added thereto, and the deposited solid was collected by filtering to thereby obtain a crude product. The obtained crude product was purified through recrystallization from a monochlorobenzene solvent to thereby obtain 3.0 g (yield: 33%) of a white powder of 4,6-bis-{4-(pyridine-3-yl)-phenyl}-2-(9,9'-spirobi[9H]fluorene-2-yl)-pyrimidine (Compound-19).

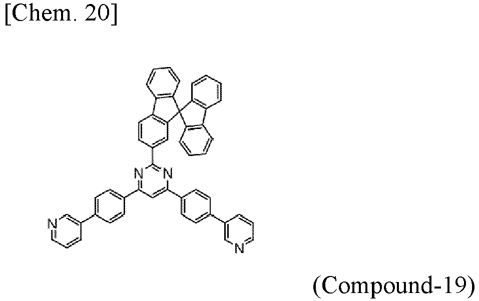

The structure of the obtained white powder was identified using NMR

In ¹H-NMR (CDCl₃), the following signals of 32 hydrogens were detected.
δ (ppm) = 8.95 (2H), 8.87 (1H), 8.66 (2H), 8.30 (4H), 8.08 (1H), 8.06 (1H), 8.01-7.90 (6H), 7.76 (4H), 7.46-7.38(5H), 7.17 (2H), 7.14 (1H), 6.83 (2H), 6.75 (1H)

### Example 4

### <Synthesis of 2-(phenanthrene-2-yl)-4-{3-(pyridine-3-yl)-phenyl}-6-{4-(pyridine-3-yl)-phenyl}-pyrimi dine (Compound-27)>

First, 8.9 g of 4-(4-chloro-phenyl)-2-(phenanthrene-2-yl)-6-{3-(pyridine-3-yl)-phenyl}-pyrimidine, 2.3 g of 3-pyridylboronic acid, 0.5 g of tris(dibenzylideneacetone)dipalladium(0), 1.0 g of tricyclohexylphosphine, and 7.3 g of tripotassium phosphate were placed in a reaction vessel, and stirred under reflux overnight in a 1,4-dioxane/H₂O mixed solvent. The reaction system was allowed to cool. Then, the reaction system was subjected to separatory operation to extract and separate an organic layer, and the organic layer was concentrated under reduced pressure. The thus obtained crude product was purified by column chromatography (carrier: silica gel, eluent: dichloromethane/ethyl acetate) to thereby obtain 6.0 g (yield: 63%) of a white powder of 2-(phenanthrene-2-yl)-4-{3-(pyridine-3-yl)-phenyl}-6-{4-(pyridine-3-yl)-phenyl}-pyrimi dine (Compound-27).

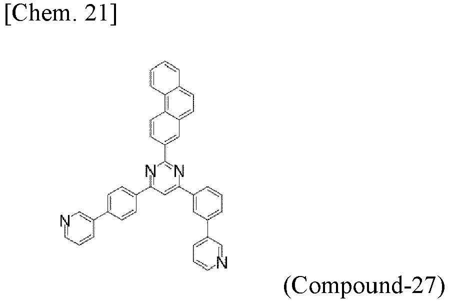

The structure of the obtained white powder was identified using NMR

In ¹H-NMR (CDCl₃), the following signals of 26 hydrogens were detected.
δ (ppm) = 9.29 (1H), 9.05 (1H), 9.01 (2H), 8.87 (1H), 8.80 (1H), 8.70 (2H), 8.53 (1H), 8.51 (2H), 8.39 (1H), 8.15 (1H), 8.10-7.93 (4H), 7.90-7.62 (7H), 7.48 (2H)

### Example 5

### <Synthesis of 4-{3-(pyridine-3-yl)-phenyl}-6-{4-(pyridine-3-yl)-phenyl}-2-(9,9'-spirobi[9H]fluorene-2 -yl)-pyrimidine (Compound-31)>

First, 11.4 g of 4-(4-chloro-phenyl)-6-{3-(pyridine-3-yl)-phenyl}-2-(9,9'-spirobi[9H]fluorene-2-yl)-pyri midine, 2.3 g of 3-pyridylboronic acid, 0.5 g of tris(dibenzylideneacetone)dipalladium(0), 0.5 g of tricyclohexylphosphine, and 7.4 g of tripotassium phosphate were placed in a reaction vessel, and stirred under reflux overnight in a 1,4-dioxane/H₂O mixed solvent. The reaction system was allowed to cool. Then, the reaction system was subjected to separatory operation to extract and separate an organic layer, and the organic layer was concentrated under reduced pressure. The thus obtained crude product was purified by column chromatography (carrier: silica gel, eluent: dichloromethane/ethyl acetate) to thereby obtain 9.2 g (yield: 76%) of a white powder of 4-{3-(pyridine-3-yl)-phenyl}-6-{4-(pyridine-3-yl)-phenyl}-2-(9,9'-spirobi[9H]fluorene-2 -yl)-pyrimidine (Compound-31).

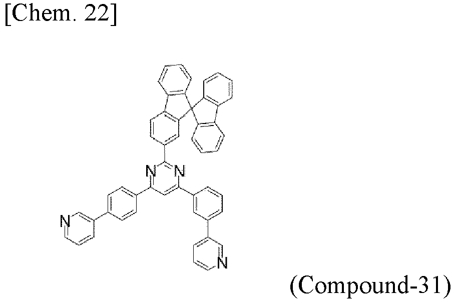

The structure of the obtained white powder was identified using NMR

In ¹H-NMR (CDCl₃), the following signals of 32 hydrogens were detected. δ (ppm) = 8.93 (2H), 8.82 (1H), 8.69 (1H), 8.65 (1H), 8.39 (1H), 8.29 (2H), 8.18 (1H), 8.05 (1H), 8.03 (1H), 7.99 (1H), 7.98-7.88(5H), 7.74 (3H), 7.65 (1H), 7.48-7.35(5H), 7.13 (3H), 6.81 (2H), 6.76 (1H)

### Example 6

### <Synthesis of 2-{4-(naphthalene-1-yl)-phenyl}-4-{4-(pyridine-3-yl)-phenyl}-6-{4-(quinoline-8-yl)-phe nyl}-pyrimidine (Compound-36)>

First, 8.0 g of 4-(4-chloro-phenyl)-6-{4-(pyridine-3-yl)-phenyl}-2-{4-(naphthalene-1-yl)-phenyl}-pyrim idine, 2.8 g of 8-quinolylboronic acid, 0.4 g of tris(dibenzylideneacetone)dipalladium(0), 0.4 g of tricyclohexylphosphine, and 6.2 g of tripotassium phosphate were placed in a reaction vessel, and stirred under reflux overnight in a 1,4-dioxane/H₂O mixed solvent. The reaction system was allowed to cool. Then, methanol was added thereto, and the deposited solid was collected by filtering to obtain a crude product. The obtained crude product was purified through recrystallization from a monochlorobenzene solvent to thereby obtain 7.0 g (yield: 74%) of a white powder of 2-{4-(naphthalene-1-yl)-phenyl}-4-{4-(pyridine-3-yl)-phenyl}-6-{4-(quinoline-8-yl)-phe nyl}-pyrimidine (Compound-36).

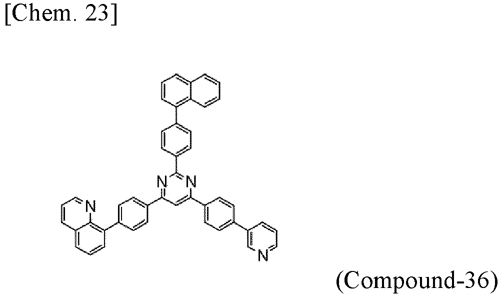

The structure of the obtained white powder was identified using NMR

In ¹H-NMR (CDCl₃), the following signals of 30 hydrogens were detected.
δ (ppm) = 9.03 (1H), 9.00 (1H), 8.93 (2H), 8.69 (1H), 8.51 (4H), 8.29 (1H), 8.20 (1H), 8.09-7.78 (10H), 7.74 (2H), 7.70 (1H), 7.65-7.41 (6H)

### Example 7

### <Synthesis of 4,6-bis-{4-(pyridine-3-yl)-phenyl}-2-(9,9'-spirobi[9H]fluorene-2-yl)-pyrimidine (Compound-84)>

First, 8.2 g of 4,6-bis-(4-chloro-phenyl)-2-(9,9'-spirobi[9H]fluorene-2-yl)-pyrimidine, 3.9 g of 3-pyridylboronic acid, 0.6 g of tris(dibenzylideneacetone)dipalladium(0), 0.7 g of tricyclohexylphosphine, and 5.5 g of potassium carbonate were placed in a reaction vessel, and stirred under reflux overnight in a 1,4-dioxane/H₂O mixed solvent. The reaction system was allowed to cool. Then, H₂O was added thereto, and the deposited solid was collected by filtering to obtain a crude product. The obtained crude product was purified through recrystallization from a monochlorobenzene solvent to thereby obtain 2.6 g (yield 28%) of a white powder of 4,6-bis-{4-(pyridine-3-yl)-phenyl}-2-(9,9'-spirobi[9H]fluorene-2-yl)-pyrimidine (Compound-84).

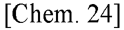

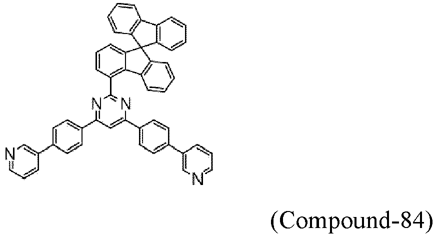

The structure of the obtained white powder was identified using NMR

In ¹H-NMR (CDCl₃), the following signals of 32 hydrogens were detected.
δ (ppm) = 8.97 (2H), 8.66 (2H), 8.49 (4H), 8.34 (1H), 7.99 (2H), 7.88 (3H), 7.82 (4H), 7.79 (1H), 7.43 (4H), 7.39 (1H), 7.16 (2H), 7.10 (2H), 6.90 (2H), 6.87 (1H), 6.76 (1H)

### Example 8

### <Synthesis of 4,6-bis-{4-(pyridine-3-yl)-phenyl}-2-(9,9-diphenyl[9H]fluorene-3-yl)-pyrimidine (Compound-85)>

First, 7.0 g of 4,6-bis-(4-chloro-phenyl)-2-(9,9-diphenyl[9H]fluorene-3-yl)-pyrimidine, 3.3 g of 3-pyridylboronic acid, 0.3 g of tris(dibenzylideneacetone)dipalladium(0), 0.3 g of tricyclohexylphosphine, and 4.8 g of tripotassium phosphate were placed in a reaction vessel, and stirred under reflux overnight in a 1,4-dioxane/H₂O mixed solvent. The reaction system was allowed to cool. Then, the reaction system was subjected to separatory operation to extract and separate an organic layer, and the organic layer was concentrated under reduced pressure. The thus obtained crude product was purified by column chromatography (carrier: silica gel, eluent: dichloromethane/ethyl acetate) to thereby obtain 2.6 g (yield: 28%) of a white powder of 4,6-bis-{4-(pyridine-3-yl)-phenyl}-2-(9,9-diphenyl[9H]fluorene-3-yl)-pyrimidine (Compound-85).

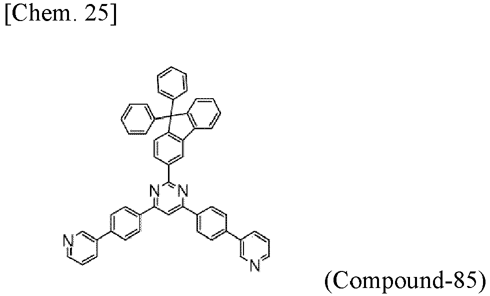

The structure of the obtained white powder was identified using NMR

In ¹H-NMR (CDCl₃), the following signals of 34 hydrogens were detected.
δ (ppm) = 9.11 (1H), 9.00 (2H), 8.70 (2H), 8.68 (1H), 8.48 (4H), 8.17 (1H), 8.03 (3H), 7.85 (4H), 7.62 (1H), 7.47 (4H), 7.38-7.21 (11H)

### Example 9

### <Synthesis of 4-{4-(isoquinoline-4-yl)-phenyl}-6-{3-(pyridine-3-yl)-phenyl}-2-(9,9'-spirobi[9H]fluore ne-2-yl)-pyrimidine (Compound-86)>

First, 11.3 g of 4-(4-chloro-phenyl)-6-{3-(pyridine-3-yl)-phenyl}-2-(9,9'-spirobi[9H]fluorene-2-yl)-pyri midine, 3.6 g of 4-isoquinolylboronic acid, 0.5 g of tris(dibenzylideneacetone)dipalladium(0), 0.5 g of tricyclohexylphosphine, and 7.3 g of tripotassium phosphate were placed in a reaction vessel, and stirred under reflux overnight in a 1,4-dioxane/H₂O mixed solvent. The reaction system was allowed to cool. Then, the reaction system was subjected to separatory operation to extract and separate an organic layer, and the organic layer was concentrated under reduced pressure. The thus obtained crude product was purified by column chromatography (carrier: silica gel, eluent: dichloromethane/ethyl acetate) to thereby obtain 8.1 g (yield: 63%) of a white powder of 4-{4-(isoquinoline-4-yl)-phenyl}-6-{3-(pyridine-3-yl)-phenyl}-2-(9,9'-spirobi[9H]fluore ne-2-yl)-pyrimidine (Compound-86).

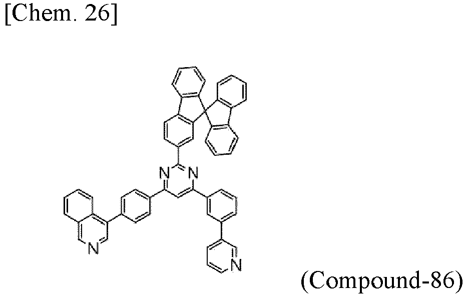

The structure of the obtained white powder was identified using NMR

In ¹H-NMR (CDCl₃), the following signals of 34 hydrogens were detected.
δ (ppm) = 9.30 (1H), 8.93 (1H), 8.84 (1H), 8.69 (1H), 8.54 (1H), 8.40 (1H), 8.33 (2H), 8.19 (1H), 8.06 (4H), 7.95 (2H), 7.92 (2H), 7.89 (1H), 7.77-7.60 (6H), 7.45 (1H), 7.41 (2H), 7.37 (1H), 7.14 (3H), 6.81 (2H), 6.75 (1H)

### Example 10

### <Synthesis of 4-{3-(pyridine-3-yl)-phenyl}-6-{4-(quinoline-8-yl)-phenyl}-2-(9,9'-spirobi[9H]fluorene-2-yl)-pyrimidine (Compound-87)>

First, 7.0 g of 4-(4-chloro-phenyl)-6-{3-(pyridine-3-yl)-phenyl}-2-(9,9'-spirobi[9H]fluorene-2-yl)-pyri midine, 2.2 g of 8-quinolylboronic acid, 0.3 g of tris(dibenzylideneacetone)dipalladium(0), 0.1 g of tricyclohexylphosphine, and 4.5 g of tripotassium phosphate were placed in a reaction vessel, and stirred under reflux overnight in a 1,4-dioxane/H₂O mixed solvent. The reaction system was allowed to cool. Then, the reaction system was subjected to separatory operation to extract and separate an organic layer, and the organic layer was concentrated under reduced pressure. The thus obtained crude product was purified by column chromatography (carrier: silica gel, eluent: dichloromethane/ethyl acetate) to thereby obtain 4.0 g (yield: 50%) of a white powder of 4-{3-(pyridine-3-yl)-phenyl}-6-{4-(quinoline-8-yl)-phenyl}-2-(9,9'-spirobi[9H]fluorene-2-yl)-pyrimidine (Compound-87).

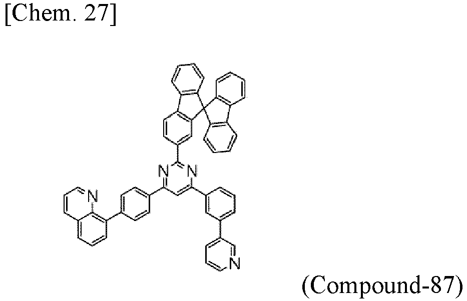

The structure of the obtained white powder was identified using NMR

In ¹H-NMR (CDCl₃), the following signals of 34 hydrogens were detected.
δ (ppm) = 9.00 (1H), 8.93 (1H), 8.84 (1H), 8.69 (1H), 8.36 (1H), 8.29 (2H), 8.24 (1H), 8.16 (1H), 8.06 (1H), 8.03 (1H), 8.02 (1H), 7.96-7.82 (7H), 7.79 (1H), 7.73 (1H), 7.65 (1H), 7.62 (1H), 7.46 (1H), 7.43 (1H), 7.38 (3H), 7.13 (3H), 6.81 (2H), 6.76 (1H)

### Example 11

### <Synthesis of 4-{3-(pyridine-3-yl)-phenyl}-6-{4-(quinoline-3-yl)-phenyl}-2-(9,9'-spirobi[9H]fluorene-2-yl)-pyrimidine (Compound-88)>

First, 7.0 g of 4-(4-chloro-phenyl)-6-{3-(pyridine-3-yl)-phenyl}-2-(9,9'-spirobi[9H]fluorene-2-yl)-pyri midine, 3.3 g of 3-quinolylboronic acid ester, 0.3 g of tris(dibenzylideneacetone)dipalladium(0), 0.1 g of tricyclohexylphosphine, and 4.5 g of tripotassium phosphate were placed in a reaction vessel, and stirred under reflux overnight in a 1,4-dioxane/H₂O mixed solvent. The reaction system was allowed to cool. Then, the reaction system was subjected to separatory operation to extract and separate an organic layer, and the organic layer was concentrated under reduced pressure. The thus obtained crude product was purified by column chromatography (carrier: silica gel, eluent: dichloromethane/ethyl acetate) to thereby obtain 5.2 g (yield: 60%) of a white powder of 4-{3-(pyridine-3-yl)-phenyl}-6-{4-(quinoline-3-yl)-phenyl}-2-(9,9'-spirobi[9H]fluorene-2-yl)-pyrimidine (Compound-88).

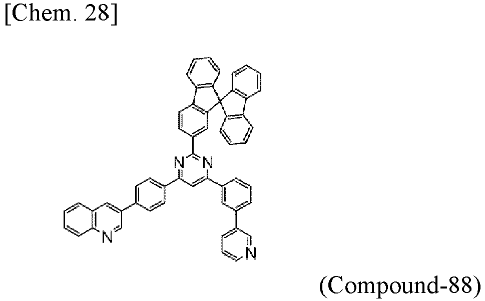

The structure of the obtained white powder was identified using NMR

In ¹H-NMR (CDCl₃), the following signals of 34 hydrogens were detected.
δ (ppm) = 9.27 (1H), 8.96 (1H), 8.86 (1H), 8.72 (1H), 8.41 (2H), 8.36 (2H), 8.22 (1H), 8.19 (1H), 8.08 (1H), 8.07 (1H), 8.04 (1H), 7.99-7.86 (7H), 7.79 (1H), 7.76 (1H), 7.67 (2H), 7.48 (1H), 7.45 (1H), 7.41 (2H), 7.16 (3H), 6.84 (2H), 6.78 (1H)

### Example 12

### <Synthesis of 4-{3-(pyridine-3-yl)-phenyl}-6-{4-(pyridine-4-yl)-phenyl}-2-(9,9'-spirobi[9H]fluorene-2 -yl)-pyrimidine (Compound-89)>

First, 7.0 g of 4-(4-chloro-phenyl)-6-{3-(pyridine-3-yl)-phenyl}-2-(9,9'-spirobi[9H]fluorene-2-yl)-pyri midine, 1.4 g of 4-pyridylboronic acid, 0.3 g of tris(dibenzylideneacetone)dipalladium(0), 0.1 g of tricyclohexylphosphine, and 4.5 g of tripotassium phosphate were placed in a reaction vessel, and stirred under reflux overnight in a 1,4-dioxane/H₂O mixed solvent. The reaction system was allowed to cool. Then, the reaction system was subjected to separatory operation to extract and separate an organic layer, and the organic layer was concentrated under reduced pressure. The thus obtained crude product was purified by column chromatography (carrier: silica gel, eluent: dichloromethane/ethyl acetate) to thereby obtain 6.4 g (yield: 85%) of a white powder of 4-{3-(pyridine-3-yl)-phenyl}-6-{4-(pyridine-4-yl)-phenyl}-2-(9,9'-spirobi[9H]fluorene-2 -yl)-pyrimidine (Compound-89).

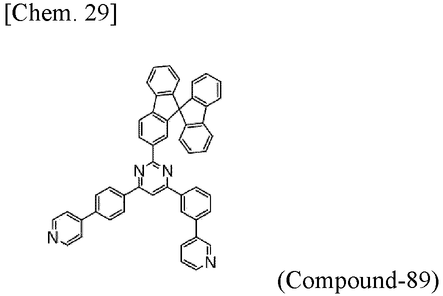

The structure of the obtained white powder was identified using NMR

In ¹H-NMR (CDCl₃), the following signals of 32 hydrogens were detected.
δ (ppm) = 8.95 (1H), 8.84 (1H), 8.73 (3H), 8.40 (1H), 8.31 (2H), 8.19 (1H), 8.07 (1H), 8.05 (1H), 8.01 (1H), 7.99-7.91 (4H), 7.81 (2H), 7.76 (1H), 7.66 (1H), 7.59 (2H), 7.49-7.37 (4H), 7.16 (3H), 6.83 (2H), 6.78 (1H)

### Example 13

### <Synthesis of 2-{4-(naphthalene-1-yl)-phenyl}-4-{4-(pyridine-3-yl)-phenyl}-6-{4-(quinoline-3-yl)-phe nyl}-pyrimidine (Compound-93)>

First, 8.0 g of 4-(4-chloro-phenyl)-6-{4-(pyridine-3-yl)-phenyl}-2-{4-(naphthalene-1-yl)-phenyl}-pyrim idine, 4.1 g of 3-quinolylboronic acid ester, 0.4 g of tris(dibenzylideneacetone)dipalladium(0), 0.4 g of tricyclohexylphosphine, and 6.2 g of tripotassium phosphate were placed in a reaction vessel, and stirred under reflux overnight in a 1,4-dioxane/H₂O mixed solvent. The reaction system was allowed to cool. Then, methanol was added thereto, and the deposited solid was collected by filtering to obtain a crude product. The obtained crude product was purified through recrystallization from a monochlorobenzene solvent to thereby obtain 4.8 g (yield: 51%) of a white powder of 2-{4-(naphthalene-1-yl)-phenyl}-4-{4-(pyridine-3-yl)-phenyl}-6-{4-(quinoline-3-yl)-phe nyl}-pyrimidine (Compound-93).

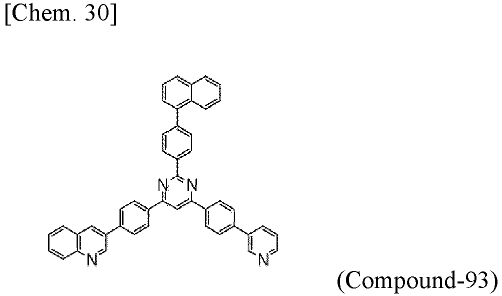

The structure of the obtained white powder was identified using NMR

In ¹H-NMR (CDCl₃), the following signals of 30 hydrogens were detected.
δ (ppm) = 9.31 (1H), 8.99 (1H), 8.91 (2H), 8.68 (1H), 8.51 (4H), 8.42 (1H), 8.20 (1H), 8.16 (1H), 8.08-7.88 (7H), 7.83 (2H), 7.79 (1H)7.74 (2H), 7.68-7.39 (6H)

### Example 14

### <Synthesis of 4-{4-(isoquinoline-4-yl)-phenyl}-2-{4-(naphthalene-1-yl)-phenyl}-6-{4-(pyridine-3-yl)-p henyl}-pyrimidine (Compound-94)>

First, 8.0 g of 4-(4-chloro-phenyl)-6-{4-(pyridine-3-yl)-phenyl}-2-{4-(naphthalene-1-yl)-phenyl}-pyrim idine, 2.8 g of 4-isoquinolylboronic acid, 0.4 g of tris(dibenzylideneacetone)dipalladium(0), 0.4 g of tricyclohexylphosphine, and 6.2 g of tripotassium phosphate were placed in a reaction vessel, and stirred under reflux overnight in a 1,4-dioxane/H₂O mixed solvent. The reaction system was allowed to cool. Then, methanol was added thereto, and the deposited solid was collected by filtering to obtain a crude product. The obtained crude product was purified through recrystallization from a monochlorobenzene solvent to thereby obtain 5.1 g (yield: 54%) of a white powder of 4-{4-(isoquinoline-4-yl)-phenyl}-2-{4-(naphthalene-1-yl)-phenyl}-6-{4-(pyridine-3-yl)-p henyl}-pyrimidine (Compound-94).

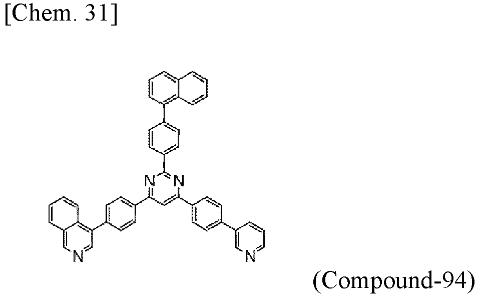

The structure of the obtained white powder was identified using NMR

In ¹H-NMR (CDCl₃), the following signals of 30 hydrogens were detected.
δ (ppm) = 9.35 (1H), 9.01 (1H), 8.93 (2H), 8.69 (1H), 8.62 (1H), 8.54 (4H), 8.22 (1H), 8.12 (1H), 8.03 (2H), 7.95 (2H), 7.86 (2H), 7.80 (2H), 7.75 (2H), 7.70 (2H), 7.65-7.42 (6H)

### Example 15

The melting point and the glass transition point of each of the pyrimidine compounds represented by the general formula (1) were measured using a high-sensitivity differential scanning calorimeter (DSC3100SA manufactured by Bruker AXS). Table 1 shows the results. In Table 1, the sign "-" means that the melting point was not found because of high amorphousness. The melting point is a measure of the vapor deposition properties, and the glass transition point (Tg) is a measure of the stability in the form of a thin film.

**Table 1**

| | Melting point | Glass transition point |
|---|---|---|
| Compound of Ex. 1 | 269°C | 136°C |
| Compound of Ex. 2 | 227°C | 113°C |
| Compound of Ex. 3 | 362°C | 165°C |
| Compound of Ex. 4 | -°C | 98°C |
| Compound of Ex. 5 | 309°C | 147°C |
| Compound of Ex. 6 | 260°C | 121°C |
| Compound of Ex. 7 | 287°C | 169°C |
| Compound of Ex. 8 | 346°C | 156°C |
| Compound of Ex. 9 | -°C | 155°C |
| Compound of Ex. 10 | 266°C | 152°C |
| Compound of Ex. 11 | 338°C | 153°C |
| Compound of Ex. 12 | 314°C | 154°C |
| Compound of Ex. 13 | -°C | 118°C |
| Compound of Ex. 14 | -°C | 123°C |

The compounds having a pyrimidine ring structure and represented by the general formula (1) had a glass transition point of at least 98°C, which means that these compounds are stable in the form of a thin film.

### Example 16

A vapor-deposited film (thickness: 100 nm) of the compound having a pyrimidine ring structure and represented by the general formula (1) was formed on an ITO substrate , and the work function was measured using an ionization potential measuring device (PYS-202 manufactured by Sumitomo Heavy Industries, Ltd.). Table 2 shows the results. The work function is a measure of the hole-transporting capability and the hole-blocking capability.

**Table 2**

| | Work function |
|---|---|
| Compound of Ex. 1 | 6.54 eV |
| Compound of Ex. 2 | 6.08 eV |
| Compound of Ex. 3 | 6.63 eV |
| Compound of Ex. 4 | 6.60 eV |
| Compound of Ex. 5 | 6.63 eV |
| Compound of Ex. 6 | 6.17 eV |
| Compound of Ex. 7 | 6.65 eV |
| Compound of Ex. 8 | 6.68 eV |
| Compound of Ex. 9 | 6.41 eV |
| Compound of Ex. 10 | 6.43 eV |
| Compound of Ex. 11 | 6.62 eV |
| Compound of Ex. 12 | 6.64 eV |
| Compound of Ex. 13 | 6.27 eV |
| Compound of Ex. 14 | 6.71 eV |

The compounds having a pyrimidine ring structure and represented by the general formula (1) had a work function value greater than 5.5 eV, which is the work function of common hole-transporting materials such as NPD and TPD. This means that these compounds have good hole-blocking capability.

### Example 17

An organic EL element was prepared by vapor-depositing, on an ITO electrode that was formed beforehand as a transparent anode 2 on a glass substrate 1, a hole-injecting layer 3, a hole-transporting layer 4, a light-emitting layer 5, a hole-blocking layer 6, an electron-transporting layer 7, an electron-injecting layer 8, and a cathode (aluminum electrode) 9 in this order, as shown in Fig. 1.

Specifically, a glass substrate 1 with an ITO film (thickness: 50 nm) formed thereon a was ultrasonically cleaned in isopropyl alcohol for 20 minutes, and then dried for 10 minutes on a hot plate heated at 200°C. After that, UV/ozone treatment was performed for 15 minutes. Then, the glass substrate with ITO was set inside a vacuum vapor deposition machine, and the pressure was reduced to 0.001 Pa or less. Subsequently, an electron acceptor (Acceptor-1) and a compound (HTM-1) having the respective structural formulae below were vapor-deposited so as to coat the transparent anode 2 through binary vapor deposition at vapor deposition rates such that the ratio of the vapor deposition rate of Acceptor-1 to that of HTM-1 was 3:97, to thereby form a hole-injecting layer 3 with a thickness of 10 nm. On this hole-injecting layer 3, a hole-transporting layer 4 (thickness: 60 nm) made of the compound (HTM-1) having the structural formula below was formed. A compound (EMD-1) and a compound (EMH-1) having the respective structural formulae below were vapor-deposited on the hole-transporting layer 4 through binary vapor deposition at vapor deposition rates such that the ratio of the vapor deposition rate of EMD-1 to that of EMH-1 was 5:95, to thereby form a light-emitting layer 5 with a thickness of 20 nm. The inventive compound of Example 1 (Compound-5) and a compound (ETM-1) having the structural formula below were vapor-deposited on this light-emitting layer 5 through binary vapor deposition at vapor deposition rates such that the ratio of the vapor deposition rate of Compound-5 to that of ETM-1 was 50:50, to thereby form a layer (thickness: 30 nm) serving as both a hole-blocking layer 6 and an electron-transporting layer 7. On this layer serving as both the hole-blocking layer 6 and the electron-transporting layer 7, an electron-injecting layer 8 (thickness: 1 nm) made of lithium fluoride was formed. Finally, aluminum was vapor-deposited to a thickness of 100 nm to thereby form a cathode 9. The prepared organic EL element was characterized in the atmosphere at normal temperature. Table 3 collectively shows the measurement results of light emission characteristics when a DC voltage was applied to the prepared organic EL element.

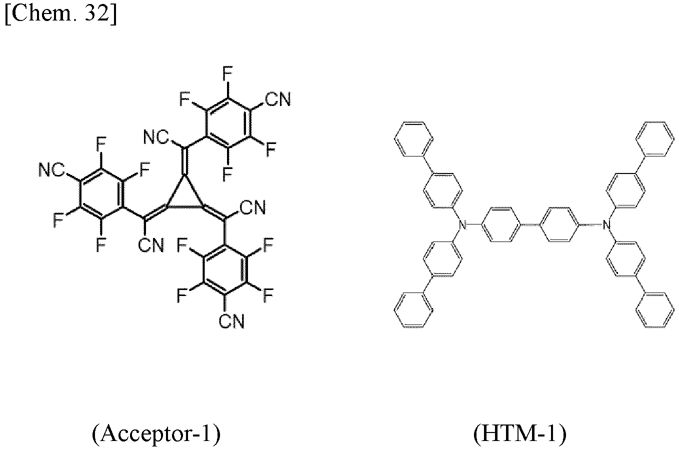

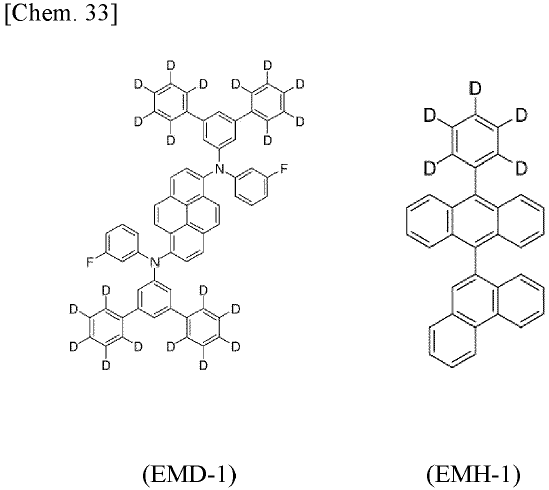

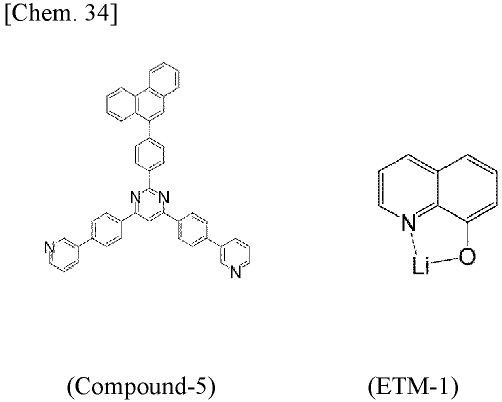

### Example 18

An organic EL element was prepared under the same conditions as in Example 17, except that, instead of the inventive compound of Example 1 (Compound-5), the compound of Example 2 (Compound-12) was used as the material of the layer serving as both the hole-blocking layer 6 and the electron-transporting layer 7, and that the binary vapor deposition was performed at vapor deposition rates such that the ratio of the vapor deposition rate of Compound-12 to that of ETM-1 was 50:50. The prepared organic EL element were characterized in the atmosphere at normal temperature. Table 3 collectively shows the measurement results of light emission characteristics when a DC voltage was applied to the prepared organic EL element.

### Example 19

An organic EL element was prepared under the same conditions as in Example 17, except that, instead of the inventive compound of Example 1 (Compound-5), the compound of Example 3 (Compound-19) was used as the material of the layer serving as both the hole-blocking layer 6 and the electron-transporting layer 7, and that the binary vapor deposition was performed at vapor deposition rates such that the ratio of the vapor deposition rate of Compound-19 to that of ETM-1 was 50:50. The prepared organic EL element was characterized in the atmosphere at normal temperature. Table 3 collectively shows the measurement results of light emission characteristics when a DC voltage was applied to the prepared organic EL element.

### Example 20

An organic EL element was prepared under the same conditions as in Example 17, except that, instead of the inventive compound of Example 1 (Compound-5), the compound of Example 4 (Compound-27) was used as the material of the layer serving as both the hole-blocking layer 6 and the electron-transporting layer 7, and that the binary vapor deposition was performed at vapor deposition rates such that the ratio of the vapor deposition rate of Compound-27 to that of ETM-1 was 50:50. The prepared organic EL element was characterized in the atmosphere at normal temperature. Table 3 collectively shows the measurement results of light emission characteristics when a DC voltage was applied to the prepared organic EL element.

### Example 21

An organic EL element was prepared under the same conditions as in Example 17, except that, instead of the inventive compound of Example 1 (Compound-5), the compound of Example 5 (Compound-31) was used as the material of the layer serving as both the hole-blocking layer 6 and the electron-transporting layer 7, and that the binary vapor deposition was performed at vapor deposition rates such that the ratio of the vapor deposition rate of Compound-31 to that of ETM-1 was 50:50. The prepared organic EL element was characterized in the atmosphere at normal temperature. Table 3 collectively shows the measurement results of light emission characteristics when a DC voltage was applied to the prepared organic EL element.

### Example 22

An organic EL element was prepared under the same conditions as in Example 17, except that, instead of the inventive compound of Example 1 (Compound-5), the compound of Example 6 (Compound-36) was used as the material of the layer serving as both the hole-blocking layer 6 and the electron-transporting layer 7, and that the binary vapor deposition was performed at vapor deposition rates such that the ratio of the vapor deposition rate of Compound-36 to that of ETM-1 was 50:50. The prepared organic EL element was characterized in the atmosphere at normal temperature. Table 3 collectively shows the measurement results of light emission characteristics when a DC voltage was applied to the prepared organic EL element.

### Example 23

An organic EL element was prepared under the same conditions as in Example 17, except that, instead of the inventive compound of Example 1 (Compound-5), the compound of Example 7 (Compound-84) was used as the material of the layer serving as both the hole-blocking layer 6 and the electron-transporting layer 7, and that the binary vapor deposition was performed at vapor deposition rates such that the ratio of the vapor deposition rate of Compound-84 to that of ETM-1 was 50:50. The prepared organic EL element was characterized in the atmosphere at normal temperature. Table 3 collectively shows the measurement results of light emission characteristics when a DC voltage was applied to the prepared organic EL element.

### Example 24

An organic EL element was prepared under the same conditions as in Example 17, except that, instead of the inventive compound of Example 1 (Compound-5), the compound of Example 8 (Compound-85) was used as the material of the layer serving as both the hole-blocking layer 6 and the electron-transporting layer 7, and that the binary vapor deposition was performed at vapor deposition rates such that the ratio of the vapor deposition rate of Compound-85 to that of ETM-1 was 50:50. The prepared organic EL element was characterized in the atmosphere at normal temperature. Table 3 collectively shows the measurement results of light emission characteristics when a DC voltage was applied to the prepared organic EL element.

### Example 25

An organic EL element was prepared under the same conditions as in Example 17, except that, instead of the inventive compound of Example 1 (Compound-5), the compound of Example 9 (Compound-86) was used as the material of the layer serving as both the hole-blocking layer 6 and the electron-transporting layer 7, and that the binary vapor deposition was performed at vapor deposition rates such that the ratio of the vapor deposition rate of Compound-86 to that of ETM-1 was 50:50. The prepared organic EL element was characterized in the atmosphere at normal temperature. Table 3 collectively shows the measurement results of light emission characteristics when a DC voltage was applied to the prepared organic EL element.

### Example 26

An organic EL element was prepared under the same conditions as in Example 17, except that, instead of the inventive compound of Example 1 (Compound-5), the compound of Example 10 (Compound-87) was used as the material of the layer serving as both the hole-blocking layer 6 and the electron-transporting layer 7, and that the binary vapor deposition was performed at vapor deposition rates such that the ratio of the vapor deposition rate of Compound-87 to that of ETM-1 was 50:50. The prepared organic EL element was characterized in the atmosphere at normal temperature. Table 3 collectively shows the measurement results of light emission characteristics when a DC voltage was applied to the prepared organic EL element.

### Example 27

An organic EL element was prepared under the same conditions as in Example 17, except that, instead of the inventive compound of Example 1 (Compound-5), the compound of Example 11 (Compound-88) was used as the material of the layer serving as both the hole-blocking layer 6 and the electron-transporting layer 7, and that the binary vapor deposition was performed at vapor deposition rates such that the ratio of the vapor deposition rate of Compound-88 to that of ETM-1 was 50:50. The prepared organic EL element was characterized in the atmosphere at normal temperature. Table 3 collectively shows the measurement results of light emission characteristics when a DC voltage was applied to the prepared organic EL element.

### Example 28

An organic EL element was prepared under the same conditions as in Example 17, except that, instead of the inventive compound of Example 1 (Compound-5), the compound of Example 12 (Compound-89) was used as the material of the layer serving as both the hole-blocking layer 6 and the electron-transporting layer 7, and that the binary vapor deposition was performed at vapor deposition rates such that the ratio of the vapor deposition rate of Compound-89 to that of ETM-1 was 50:50. The prepared organic EL element was characterized in the atmosphere at normal temperature. Table 3 collectively shows the measurement results of light emission characteristics when a DC voltage was applied to the prepared organic EL element.

### Example 29

An organic EL element was prepared under the same conditions as in Example 17, except that, instead of the inventive compound of Example 1 (Compound-5), the compound of Example 13 (Compound-93) was used as the material of the layer serving as both the hole-blocking layer 6 and the electron-transporting layer 7, and that the binary vapor deposition was performed at vapor deposition rates such that the ratio of the vapor deposition rate of Compound-93 to that of ETM-1 was 50:50. The prepared organic EL element was characterized in the atmosphere at normal temperature. Table 3 collectively shows the measurement results of light emission characteristics when a DC voltage was applied to the prepared organic EL element.

### Example 30

An organic EL element was prepared under the same conditions as in Example 17, except that, instead of the inventive compound of Example 1 (Compound-5), the compound of Example 14 (Compound-94) was used as the material of the layer serving as both the hole-blocking layer 6 and the electron-transporting layer 7, and that the binary vapor deposition was performed at vapor deposition rates such that the ratio of the vapor deposition rate of Compound-94 to that of ETM-1 was 50:50. The prepared organic EL element was characterized in the atmosphere at normal temperature. Table 3 collectively shows the measurement results of light emission characteristics when a DC voltage was applied to the prepared organic EL element.

### Comparative Example 1

For comparison, an organic EL element was prepared under the same conditions as in Example 17, except that, instead of the inventive compound of Example 1 (Compound-5), a compound having the structural formula below (ETM-2) (see Patent Literature 6, for example) was used as the material of the layer serving as both the hole-blocking layer 6 and the electron-transporting layer 7, and that the binary vapor deposition was performed at vapor deposition rates such that the ratio of the vapor deposition rate of ETM-2 to that of ETM-1 was 50:50. The prepared organic EL element was characterized in the atmosphere at normal temperature. Table 3 collectively shows the measurement results of light emission characteristics when a DC voltage was applied to the prepared organic EL element.

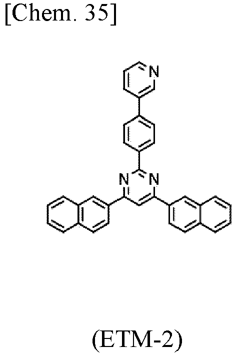

### Comparative Example 2

For comparison, an organic EL element was prepared under the same conditions as in Example 17, except that, instead of the inventive compound of Example 1 (Compound-5), a compound having the structural formula below (ETM-3) (see Patent Literature 8, for example) was used as the material of the layer serving as both the hole-blocking layer 6 and the electron-transporting layer 7, and that the binary vapor deposition was performed at vapor deposition rates such that the ratio of the vapor deposition rate of ETM-3 to that of ETM-1 was 50:50. The prepared organic EL element was characterized in the atmosphere at normal temperature. Table 3 collectively shows the measurement results of light emission characteristics when a DC voltage was applied to the prepared organic EL element.

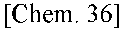

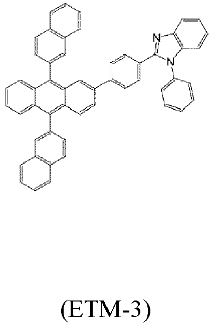

### Comparative Example 3

For comparison, an organic EL element was prepared under the same conditions as in Example 17, except that, instead of the inventive compound of Example 1 (Compound-5), a compound having the structural formula below (ETM-4) (see Patent Literature 9, for example) was used as the material of the layer serving as both the hole-blocking layer 6 and the electron-transporting layer 7, and that the binary vapor deposition was performed at vapor deposition rates such that the ratio of the vapor deposition rate of ETM-4 to that of ETM-1 was 50:50. The prepared organic EL element was characterized in the atmosphere at normal temperature. Table 3 collectively shows the measurement results of light emission characteristics when a DC voltage was applied to the prepared organic EL element.

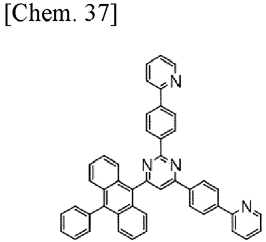

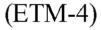

### Comparative Example 4

For comparison, an organic EL element was prepared under the same conditions as in Example 17, except that, instead of the inventive compound of Example 1 (Compound-5), a compound having the structural formula below (ETM-5) (see Patent Literature 10, for example) was used as the material of the layer serving as both the hole-blocking layer 6 and the electron-transporting layer 7, and that the binary vapor deposition was performed at vapor deposition rates such that the ratio of the vapor deposition rate of ETM-5 to that of ETM-1 was 50:50. The prepared organic EL element was characterized in the atmosphere at normal temperature. Table 3 collectively shows the measurement results of light emission characteristics when a DC voltage was applied to the prepared organic EL element.

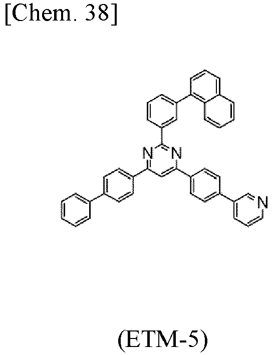

The element lifespan of each of the organic EL elements prepared in Examples 17 to 30 and Comparative Examples 1 to 4 was measured. Table 3 collectively shows the results. The element lifespan was determined as follows: the organic EL element was driven by constant current to emit light at an initial luminance (luminance when light emission started) of 2,000 cd/m², and the time taken for the luminance to decay to 1,900 cd/m² (corresponding to 95% based on the initial luminance (100%): 95% decay) was determined and defined as the element lifespan.

**Table 3**

| | Layer serving as both hole-blocking layer and electron -transporti ng layer | Voltage [V] (@10mA/cm²) | Luminance [cd/m²] (@10mA/cm²) | Luminous efficacy [cd/A] (@10mA/cm²) | Power efficiency [lm/W] (@10mA/cm²) | Element lifespan (95% decay) |
|---|---|---|---|---|---|---|
| Ex. 17 | Compound-5/ ETM-1 | 3.48 | 861 | 8.62 | 7.80 | 264 hrs. |
| Ex. 18 | Compound-12/ ETM-1 | 3.59 | 850 | 8.50 | 7.44 | 248 hrs. |
| Ex. 19 | Compound-19/ ETM-1 | 3.50 | 823 | 8.21 | 7.38 | 293 hrs. |
| Ex. 20 | Compound-27/ ETM-1 | 3.50 | 887 | 8.89 | 7.98 | 262 hrs. |
| Ex. 21 | Compound-31/ ETM-1 | 3.54 | 876 | 8.77 | 7.79 | 282 hrs. |
| Ex. 22 | Compound-36/ ETM-1 | 3.41 | 898 | 8.99 | 8.27 | 244 hrs. |
| Ex. 23 | Compound-84/ ETM-1 | 3.35 | 873 | 8.72 | 8.17 | 253 hrs. |
| Ex. 24 | Compound-85/ ETM-1 | 3.51 | 830 | 8.29 | 7.43 | 290 hrs. |
| Ex. 25 | Compound-86/ ETM-1 | 3.33 | 874 | 8.73 | 8.24 | 276 hrs. |
| Ex. 26 | Compound-87/ ETM-1 | 3.39 | 897 | 8.97 | 8.32 | 239 hrs. |
| Ex. 27 | Compound-88/ ETM-1 | 3.31 | 860 | 8.58 | 8.15 | 268 hrs. |
| Ex. 28 | Compound-89/ ETM-1 | 3.49 | 832 | 8.32 | 7.50 | 288 hrs. |
| Ex. 29 | Compound-93/ ETM-1 | 3.34 | 883 | 8.82 | 8.30 | 259 hrs. |
| Ex. 30 | Compound-94/ ETM-1 | 3.53 | 860 | 8.61 | 7.68 | 261 hrs. |
| Com. Ex. 1 | ETM-2/ ETM-1 | 3.82 | 805 | 8.05 | 6.62 | 165 hrs. |
| Com. Ex. 2 | ETM-3/ ETM-1 | 4.01 | 659 | 6.59 | 5.16 | 203 hrs. |
| Com. Ex. 3 | ETM-4/ ETM-1 | 3.72 | 763 | 7.62 | 6.44 | 189 hrs. |
| Com. Ex. 4 | ETM-5/ ETM-1 | 3.83 | 692 | 6.92 | 5.68 | 194 hrs. |

As shown in Table 1, a current of 10 mA/cm² in terms of a current density was passed through the organic EL elements, and at that time, while the organic EL elements of Comparative Examples 1 to 4 had a driving voltage of 3.72 to 4.01 V, the organic EL elements of Examples 17 to 30 had a lower driving voltage of 3.31 to 3.59 V.

While the organic EL elements of Comparative Examples 1 to 4 had a luminous efficacy of 6.59 to 8.05 cd/A, the organic EL elements of Examples 17 to 30 had an improved luminous efficacy of 8.21 to 8.99 cd/A.

While the organic EL elements of Comparative Examples 1 to 4 had a power efficiency of 5.16 to 6.62 lm/W, the organic EL elements of Examples 17 to 30 had a significantly improved power efficiency of 7.38 to 8.32 lm/W.

While the organic EL elements of Comparative Examples 1 to 4 had an element lifespan (95% decay) of 165 to 203 hours, the organic EL elements of Examples 17 to 30 had a significantly longer lifespan of 239 to 293 hours.

As described above, the organic EL elements of the present invention had excellent luminous efficacy and power efficiency as well as a long lifespan.

### Industrial Applicability

A compound having a specific pyrimidine ring structure of the present invention has good electron-injecting properties and excellent hole-blocking capability and is stable in the form of a thin film, and the compound of the present invention is therefore an excellent compound for an organic EL element. An organic EL element prepared by using this compound can achieve high efficiency and also achieve a reduced driving voltage and hence improved durability. Thus, the organic EL element can be applied to uses such as home electric appliances and lighting equipment.

## Claims

1. A compound having a pyrimidine ring structure and represented by the general formula (a-1): 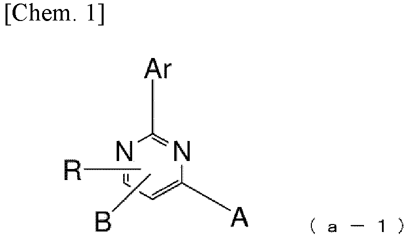 where A and B are the same or different from each other, and each are represented by the structural formula (b-1);
Ar represents a substituted or unsubstituted aromatic hydrocarbon group, a substituted or unsubstituted aromatic heterocyclic group, or a substituted or unsubstituted fused polycyclic aromatic group; and
R represents a hydrogen atom, a deuterium atom, a fluorine atom, a chlorine atom, a cyano group, a nitro group, a trimethylsilyl group, a triphenylsilyl group, a substituted or unsubstituted aromatic hydrocarbon group, a substituted or unsubstituted aromatic heterocyclic group, a substituted or unsubstituted fused polycyclic aromatic group, a linear or branched alkyl group having 1 to 6 carbon atoms and optionally having a substituent, a cycloalkyl group having 5 to 10 carbon atoms and optionally having a substituent, a linear or branched alkenyl group having 2 to 6 carbon atoms and optionally having a substituent, a linear or branched alkyloxy group having 1 to 6 carbon atoms and optionally having a substituent, or a cycloalkyloxy group having 5 to 10 carbon atoms and optionally having a substituent,
[Chem. 2]
---L-Het (b - 1)
where L represents a single bond, or a divalent group of a substituted or unsubstituted aromatic hydrocarbon group, a substituted or unsubstituted aromatic heterocyclic group, or a substituted or unsubstituted fused polycyclic aromatic group;
Het represents a substituted or unsubstituted pyridyl group, a substituted or unsubstituted pyrimidyl group, a substituted or unsubstituted triazyl group, a substituted or unsubstituted quinolyl group, a substituted or unsubstituted isoquinolyl group, a substituted or unsubstituted phenanthrolinyl group, a substituted or unsubstituted indolyl group, a substituted or unsubstituted azafluorenyl group, a substituted or unsubstituted diazafluorenyl group, a substituted or unsubstituted azaspirobifluorenyl group, or a substituted or unsubstituted diazaspirobifluorenyl group; and
the dashed line represents a binding site to the pyrimidine ring.

2. The compound having a pyrimidine ring structure as set forth in claim 1, wherein the compound is represented by the general formula (a-2): 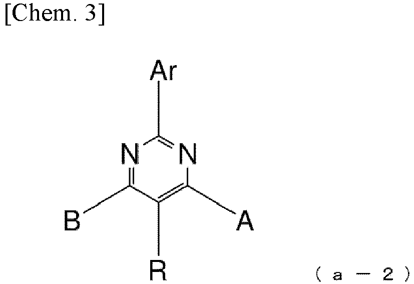 where A, B, Ar, and R are the same as defined in the general formula (a-1).

3. The compound having a pyrimidine ring structure as set forth in claim 1, wherein the compound is represented by the general formula (a-3): 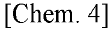 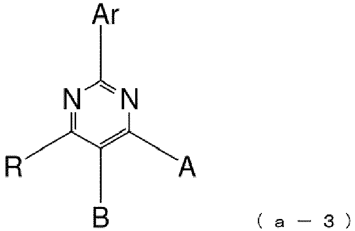 where A, B, Ar, and R are the same as defined in the general formula (a-1).

4. The compound having a pyrimidine ring structure as set forth in claim 2,
wherein A and B are different from each other.

5. The compound having a pyrimidine ring structure as set forth in claim 3,
wherein A and B are different from each other.

6. The compound having a pyrimidine ring structure as set forth in claim 4,
wherein L in the structural formula (b-1) represents a single bond, a substituted or unsubstituted phenylene group, a substituted or unsubstituted naphthylene group, a substituted or unsubstituted anthranylene group, a substituted or unsubstituted phenanthrenylene group, or a substituted or unsubstituted biphenylene group.

7. The compound having a pyrimidine ring structure as set forth in claim 5,
wherein L in the structural formula (b-1) represents a single bond, a substituted or unsubstituted phenylene group, a substituted or unsubstituted naphthylene group, a substituted or unsubstituted anthranylene group, a substituted or unsubstituted phenanthrenylene group, or a substituted or unsubstituted biphenylene group.

8. The compound having a pyrimidine ring structure as set forth in claim 6,
wherein Het in the structural formula (b-1) represents an unsubstituted pyridyl group, an unsubstituted quinolyl group, an unsubstituted isoquinolyl group, or an unsubstituted phenanthrolinyl group.

9. The compound having a pyrimidine ring structure as set forth in claim 7,
wherein Het in the structural formula (b-1) represents an unsubstituted pyridyl group, an unsubstituted quinolyl group, an unsubstituted isoquinolyl group, or an unsubstituted phenanthrolinyl group.

10. An organic electroluminescent element comprising a pair of electrodes and one or more organic layers sandwiched there between,
wherein the compound having a pyrimidine ring structure as set forth in any one of claims 1 to 9 is included in at least one of the organic layers as a constituent material thereof.

11. The organic electroluminescent element as set forth in claim 10,
wherein the organic layer including the compound having a pyrimidine ring structure is an electron-transporting layer.

12. The organic electroluminescent element as set forth in claim 10,
wherein the organic layer including the compound having a pyrimidine ring structure is a hole-blocking layer.

13. The organic electroluminescent element as set forth in claim 10,
wherein the organic layer including the compound having a pyrimidine ring structure is a light-emitting layer.

14. The organic electroluminescent element as set forth in claim 10,
wherein the organic layer including the compound having a pyrimidine ring structure is an electron-injecting layer.
